(19) Europäisches Patentamt
European Patent Office
Office européen des brevets

(11) EP 1 347 060 A1

(12) **EUROPEAN PATENT APPLICATION**
published in accordance with Art. 158(3) EPC

(43) Date of publication:
**24.09.2003 Bulletin 2003/39**

(21) Application number: **01272324.3**

(22) Date of filing: **26.12.2001**

(51) Int Cl.7: **C12Q 1/68**, C12N 15/09

(86) International application number:
**PCT/JP01/11422**

(87) International publication number:
**WO 02/052043 (04.07.2002 Gazette 2002/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **26.12.2000 JP 2000396222**
**26.12.2000 JP 2000396321**
**29.06.2001 JP 2001199552**
**13.09.2001 JP 2001278920**

(83) **Declaration under Rule 28(4) EPC (expert solution)**

(71) Applicant: **TAKARA BIO INC.**
**Otsu-shi, Shiga 520-2193 (JP)**

(72) Inventors:
- **SHIMADA, Masamitsu**
  **Otsu-shi, Shiga 520-2143 (JP)**
- **HINO, Fumitsugu**
  **Kusatsu-shi, Shiga 525-0028 (JP)**
- **KATO, Ikunoshin**
  **Uji-shi, Kyoto 611-0028 (JP)**

(74) Representative: **Grund, Martin, Dr. et al**
**Dr. Volker Vossius,**
**Patentanwaltskanzlei,**
**Geibelstrasse 6**
**81679 München (DE)**

(54) **METHOD OF DETECTING PATHOGENIC MICROORGANISM**

(57) Oligonucleotide probes and primers useful in detecting pathogenic microorganisms; a method of detecting a pathogenic microorganism by using the same; and kits for this method.

EP 1 347 060 A1

## Description

Technical Field

The present invention relates to an oligonucleotide probe or primer useful for detection of a pathogenic microorganism, a method for detecting a pathogenic microorganism using the same, and a kit for the method.

Background Art

A method in which a protein derived from a pathogenic microorganism is detected by an immunological means, and a method in which a specific region of a gene derived from a pathogenic microorganism is detected, for example, after amplifying the region using a nucleic acid amplification reaction are known as methods for detecting a pathogenic microorganism. Nucleic acid amplification reactions include: the polymerase chain reaction (PCR) method (United States Patent Nos. 4,683,195, 4,683,202 and 4,800,159); the reverse transcription-PCR (RT-PCR) method, which is a combination of the PCR method and a reverse transcriptase reaction (Trends in Biotechnology, 10:146-152 (1992)); the ligase chain reaction (LCR) method (EP 320,308 published on June 14, 1989); and the transcription-based amplification system (TAS) method (PCR Protocols, Academic Press Inc., 1990, pp. 245-252).

Nucleic acid amplification methods that can be carried out under isothermal conditions have been developed. Examples thereof include: the strand displacement amplification (SDA) method (JP-B 7-114718); the self-sustained sequence replication (3SR) method; the nucleic acid sequence based amplification (NASBA) method (Japanese Patent No. 2650159); the transcription-mediated amplification (TMA) method; the Qβ replicase method (Japanese Patent No. 2710159); and the various modified SDA methods (United States Patent No. 5,824,517, WO 99/09211, WO 95/25180 and WO 99/49081). A method for enzymatically synthesizing an oligonucleotide under isothermal conditions is described in United States Patent No. 5,916,777. Furthermore, the loop-mediated isothermal amplification (LAMP) method (WO 00/28082) and the isothermal and chimeric primer-initiated amplification of nucleic acids (ICAN) method (WO 00/56877) are known.

As described above, various nucleic acid amplification methods can be utilized in a method for detecting a pathogenic microorganism. However, it has been difficult to accomplish the detection sensitivity required by a practical test for a pathogenic microorganism by selecting a region in a target nucleic acid suitable for the nucleic acid amplification reaction, a primer suitable for the nucleic acid amplification method and a probe suitable for a target nucleic acid detection method carried out following the nucleic acid amplification method. Furthermore, a method for detecting a pathogenic microorganism that can provide reproducible results at low running costs has been desired. A Mycobacterium tuberculosis complex, a gonococcus, a chlamydia and hepatitis C virus (HCV) as examples of pathogenic microorganisms are described hereinafter.

(a) Mycobacterium tuberculosis complex

Tuberculosis is a disease that has been ranked high among causes of death of humans. There are still many patients although various therapies have been developed to date.

Tuberculosis is conventionally diagnosed using a cultivation method or a smear method. Since a Mycobacterium tuberculosis complex grows slowly, it takes one to eight weeks (usually about one month) to obtain test results. Therefore, the conventional method is not rapid. Furthermore, its accuracy is 70% or less. Thus, the method is not a satisfactory diagnosis method. Under the circumstances, rapid and accurate methods for direct detection from a sputum or the like using a Mycobacterium tuberculosis complex gene as a target, and reagents for the methods have been developed recently. Mycobacterium tuberculosis complex include Mycobacterium tuberculosis, Mycobacterium bovis BCG, Mycobacterium africanum, Mycobacterium microti and Mycobacterium canetti.

For example, a method for rapidly detecting a Mycobacterium tuberculosis complex by the PCR method using a DNA extracted from a test sample such as a sputum is described in Lancet, No. 8671, pp. 1069 (1989). In the method, a gene encoding Mycobacterium tuberculosis complex 65 kDa antigen as a target is amplified by the PCR method and detected by electrophoresis. A kit for detection of an amplification product obtained by amplifying 16S ribosomal RNA using a chemiluminescent probe is commercially available from Gen-Probe. A kit for PCR in which a DNA encoding 16S ribosomal RNA is used as a target is also commercially available from Roche.

A sequence of a gene called IS 6110 which is specific for a Mycobacterium tuberculosis complex was published (Nucleic Acids Research, 18:188 (1990)), and it is described that the gene may be useful as a probe for detection of a Mycobacterium tuberculosis complex. In fact, a method for detecting IS 6110 gene using the PCR method has been reported (J. Clin. Microbiol., 28:2668 (1990)). Thereafter, the PCR method using IS 6110 gene as a target and the usefulness of the gene have been described in many literatures. Detection of IS 6110 gene using a nucleic acid amplification method other than the PCR method such as the SDA method (Japanese Patent No. 2814422) or the LCR method (JP-A 10-500023) has been described.

However, since the above-mentioned method for detecting a Mycobacterium tuberculosis complex is not satisfactory for practical clinical tests, further improvements have been desired. For example, nucleic acid amplification-detection methods using ribosomal RNA gene as a target have been reported in many literatures. In such a method, a portion common among bacteria belonging to genus Mycobacterium is amplified and a Mycobacterium tuberculosis complex is then identified using a species-specific probe. Since the specificity for the Mycobacterium tuberculosis complex depends only on the sequence of the probe according to the method, the reliability of the method including the detection sensitivity is insufficient. Furthermore, failure in detection according to the method has been reported in many cases determined to be positive according to the cultivation method. The PCR method in which IS 6110 gene specific for a Mycobacterium tuberculosis complex is used as a target in order to increase the specificity and the detection sensitivity has been reported. However, a comparative study on tests using IS 6110 gene as a target in seven facilities has demonstrated that the test results obtained using the method greatly vary among facilities (false positive: 3-77%; sensitivity: 2-90%) (J. Clin. Microbiol., 32:277 (1994)).

The method of nucleic acid extraction from a test sample is complicated, and is insufficiently safe in view of a risk of infection to a tester.

Hybridization is utilized in the method of detection of an amplification product. A method is which an amplified double-stranded nucleic acid is first denatured in a liquid phase using a strong alkali and then the single-stranded nucleic acid resulting from the denaturation is subjected to hybridization with a probe under neutral conditions in the presence of the peeled complementary nucleic acid is conventionally used (instructions attached to Amplicor kit from Roche). According to this method, there has been a problem that the sensitivity and the specificity are not increased because the complementary amplified nucleic acid peeled from the double-stranded nucleic acid interferes with the hybridization of the probe in a competitive manner. In other words, the amplified double-stranded DNA is converted into single strands by alkali-denaturation, neutralized and then subjected to hybridization with the probe under neutral conditions in the method. Alternatively, heat denaturation may be carried out avoiding the alkali denaturation. In this case, it is impossible to precisely examine a large number of test samples. As a result, many steps are required, one of the DNA strands converted into single strands hybridizes to the probe in a competitive manner, and the sensitivity of detection of the target nucleic acid is reduced.

Since tuberculosis is an infectious disease, immediate diagnosis and measures such as quarantine of the patient are required. However, since a period of about six hours or longer is required for the procedure from collection of a test sample to reporting of results according to the conventional method or using a commercially available kit, the reporting of results and the corresponding measures to the patients are carried out not on the day of the collection of the test sample but on the next day. Consequently, a serious problem on public health that one cannot minimize the spread of tuberculosis infection remains to be solved.

The published information as described above shows that there is no method for dealing with a Mycobacterium tuberculosis complex that is reliable and useful for practical medicine.

(b) Gonococcus

A gonococcus (or Neisseria gonorrhoeae) is the pathogen of gonorrhea which is one of the most commonly reported bacterial infections in the U.S.A. Detection of N. gonorrhoeae using a DNA probe derived from a genomic fragment containing an open reading frame (ORF1) which is significantly homologous to the sequence of N. gonorrhoeae cytosine DNA. methyltransferase gene (M. Ngo PII) is described in Miyada and Born, 1991, Mol. Cell. Probes, 5:327-335; United States Patent No. 5,256,536; and United States Patent No. 5,525,717. However, no method that can be used to detect a gonococcus specifically and with a sufficient sensitivity was known.

A case of a gonococcus-infected patient also infected with Chlamydia trachomatis has been reported.

(c) Chlamydia

"A chlamydia" refers to a microorganism belonging to genus Chlamydia. Three species of genus Chlamydia, C. trachomatis, C. psittaci and C. pneumoniae, are clinically important because they can infect humans to cause diseases. Among these, the infection with Chlamydia trachomatis, which causes genital infections in both males and females, has been reported as the most common sexual infection in the advanced society. Thus, a method that can be used to specifically and timely detect Chlamydia trachomatis and a reagent for the method are needed.

(d) HCV

Conventionally, detection of HCV in a sample such as a serum is carried out using a reverse transcription-PCR (RT-PCR) method. This method comprises ten steps as follows: (1) extraction of an RNA for HCV from a serum; (2) synthesis of a cDNA using the extracted RNA as a template; (3) amplification by a PCR method in which the temperature is adjusted up and down; (4) hybridization with an immobilized probe; (5) removal of unreacted reagents by washing; (6) reaction with a labeled reagent; (7) removal of unreacted reagents by washing; (8) addition of a color-developing reagent; (9) addition of a reagent for stopping color development; and (10) measurement of absorbance. Detection of HCV by a homogeneous detection method using a real-time detection PCR method has been examined (J. Virol. Methods, Vol.64, pp. 147-159 (1997)).

Determination of HCV for a large number of test samples with a high sensitivity, conveniently, rapidly and at a low cost is very important in view of quality control of a blood preparation, or blood for transfusion or donated blood, judgement on therapeutic progress, monitoring of a disease case, or reduction in a cost for treatment. A complicated and exaggerated measurement instrument is needed for strict temperature control required for the PCR method, while the current maximal throughput is 96 test samples in five hours at the most. Then, it is impossible to measure a large number of test samples (1000 test samples or more in two hours required in a blood center or a clinical laboratory test center).

Since conventional methods have various problems as described above, a convenient technique for measuring HCV nucleic acids in a number of a test sample in a defined short period of time has been desired.

Objects of Invention

The main object of the present invention is to provide a means for measuring a pathogenic microorganism for a number of test samples with a high sensitivity, accurately, conveniently, in a defined short period of time and at a low cost.

Summary of Invention

As a result of intensive studies, the. present inventors have found a method for detecting a pathogenic microorganism utilizing a nucleic acid amplification method which is superior to the conventional nucleic acid amplification method. Furthermore, the present inventors have found a chimeric oligonucleotide primer for amplifying a target nucleic acid and a probe for detecting a target nucleic acid used for the method. Additionally, the present inventors have constructed a kit for the method. Thus, the present invention has been completed.

The first aspect of the present invention relates to a probe containing the nucleotide sequence of SEQ ID NO: 39 or a part thereof, or consisting of the nucleotide sequence of SEQ ID NO:11, which can be used to detect a Mycobacterium tuberculosis complex, i.e., Mycobacterium tuberculosis, Mycobacterium bovis BCG, Mycobacterium africanum, Mycobacterium microti and/or Mycobacterium canetti.

The second aspect of the present invention relates to a probe containing the nucleotide sequence of SEQ ID NO:27 or a part thereof, or consisting of the nucleotide sequence of SEQ ID NO:21, which can be used to detect a gonococcus Neisseria gonorrhoeae.

The third aspect of the present invention relates to a probe containing the nucleotide sequence of SEQ ID NO:22 or a part thereof, or consisting of the nucleotide sequence of SEQ ID NO:20, which can be used to detect a chlamydia Chlamydia trachomatis.

The fourth aspect of the present invention relates to a probe consisting of the nucleotide sequence of SEQ ID NO:34 or 35, which can be used to detect HCV.

The fifth aspect of the present invention relates to a probe that can hybridize to a target nucleic acid from a pathogenic microorganism at alkaline pH. According to the fifth aspect, a probe which can hybridize to a target nucleic acid from a pathogenic microorganism at alkaline pH of 8 to 14 is preferable, and the pathogenic microorganism is preferably a Mycobacterium tuberculosis complex, a gonococcus, a chlamydia or HCV. The target nucleic acid from a pathogenic microorganism is selected from a nucleotide sequence of IS 6110 gene from a Mycobacterium tuberculosis complex, a nucleotide sequence of cppB gene from a gonococcus, a nucleotide sequence of pLGV440 from a chlamydia or a nucleotide sequence of the 5' untranslated region from HCV, and a probe capable of hybridizing to the gene can be preferably used. Such a probe is exemplified by the following: a probe which contains the nucleotide sequence of SEQ ID NO:39 or a part thereof, wherein the nucleotide sequence of SEQ ID NO:39 is present in IS 6110 gene from a Mycobacterium tuberculosis complex, preferably a probe which consists of the nucleotide sequence of SEQ ID NO: 11; a probe which contains the nucleotide sequence of SEQ ID NO:27 or a part thereof, wherein the nucleotide sequence of SEQ ID NO:27 is present in cppB gene from a gonococcus, preferably a probe which consists of the nucleotide sequence of SEQ ID NO:21; a probe which contains the nucleotide sequence of SEQ ID NO:22 or a part thereof, wherein the nucleotide sequence of SEQ ID NO:22 is present in pLGV440 from a chlamydia, preferably a probe which consists of the nucleotide sequence of SEQ ID NO:20; or a probe which contains the nucleotide sequence of SEQ ID NO:34 or 35 or a part thereof, wherein the nucleotide sequences of SEQ ID NO:34 and 35 are present in the 5' untranslated region from HCV, preferably a probe which consists of the nucleotide sequence of SEQ ID NO:34 or 35.

The probe of the first to fifth aspect may be labeled. The probe may be a probe for detecting a pathogenic microorganism which is an oligonucleotide having a nucleotide sequence of continuous 8 to 53 nucleotides from the nucleotide sequence of SEQ ID NO:11, 20, 21, 34 or 35, and which is fluorescence-labeled such that the fluorescence intensity is not suppressed if the probe is hybridized to the target nucleic acid, and the fluorescence intensity is suppressed if the probe is not hybridized to the target nucleic acid. The probe may be a probe for detecting a pathogenic microorganism which consists of a sequence of continuous 8 or more nucleotides from the nucleotide sequence of SEQ ID NO:11, 20, 21, 34 or 35, or which is labeled with a rhodamine-type fluorescent dye or an oxazine-type fluorescent dye at the 5' end, and which consists of a sequence of continuous 8 or more nucleotides from the nucleotide sequence of SEQ ID NO:34. The probe may be a probe for detecting a pathogenic microorganism which has a reporter fluorescent dye and a quencher dye as fluorescent dyes for labeling, and which consists of a sequence of continuous 8 or more nucleotides from the nucleotide sequence of SEQ ID NO:11, 20, 21, 34 or 35. The reporter dye may be a fluorescein-type dye and the quencher dye may be a DABCYL-type dye. A probe which has a label selected from the group consisting of a fluorescent substance, a dye, an enzyme, biotin, gold colloid and a radioisotope can be preferably used.

The sixth aspect of the present invention relates to a method for detecting a pathogenic microorganism, the method comprising conducting hybridization of the probe of the first to fifth aspect to a target nucleic acid from a pathogenic microorganism. According to the sixth aspect, the hybridization to a target nucleic acid from a pathogenic microorganism can be conducted at alkaline pH. The pathogenic microorganism is exemplified by a Mycobacterium tuberculosis complex, a gonococcus, a chlamydia or HCV. If the pathogenic microorganism is a Mycobacterium tuberculosis complex, the target nucleic acid is preferably IS 6110 gene or a fragment thereof. Hybridization of the probe of the first or fifth aspect to amplified IS 6110 gene from a Mycobacterium tuberculosis complex and/or a fragment thereof is preferably conducted. Preferably, IS 6110 gene from a Mycobacterium tuberculosis complex and/or a fragment thereof is amplified using a primer having the nucleotide sequence of SEQ ID NO:36 or 37 or a sequence partially overlapping with said sequence. If the pathogenic microorganism is a gonococcus, the target nucleic acid is preferably cppB gene or a fragment thereof. Hybridization of the probe of the second or fifth aspect to amplified cppB gene from a gonococcus and/or a fragment thereof is preferably conducted. Preferably, cppB gene from a gonococcus and/or a fragment thereof is amplified using a primer having the nucleotide sequence of SEQ ID NO:28 or 29 or a sequence partially overlapping with said sequence. If the pathogenic microorganism is a chlamydia, the target nucleic acid is preferably pLGV440 or a fragment thereof. Hybridization of the probe of the third or fifth aspect to amplified pLGV440 from a chlamydia and/or a fragment thereof is preferably conducted. Preferably, pLGV440 from a chlamydia and/or a fragment thereof is amplified using a primer having a nucleotide sequence of any one of SEQ ID NOS:23 to 26 or a sequence partially overlapping with said sequence. If the pathogenic microorganism is HCV, the target nucleic acid is preferably the 5' untranslated region from HCV or a fragment thereof. Hybridization of the probe of the fourth or fifth aspect to amplified the 5' untranslated region from HCV and/or a fragment thereof is preferably conducted. Preferably, the 5' untranslated region from HCV and/or a fragment thereof is amplified using a primer having a nucleotide sequence of any one of SEQ ID NOS:30 to 33 or a sequence partially overlapping with said sequence.

The seventh aspect of the present invention relates to a method for detecting a pathogenic microorganism, the method comprising detecting a nucleic acid from a Mycobacterium tuberculosis complex, a gonococcus, a chlamydia or HCV using the method for detecting a target nucleic acid from a pathogenic microorganism of the sixth aspect.

The eighth aspect of the present invention relates to a method for detecting a pathogenic microorganism, the method comprising detecting a nucleic acid amplified according to a nucleic acid amplification method which comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and

(b) incubating the reaction mixture for a sufficient time to generate a reaction product. According to the eighth aspect, it is preferable that the reaction mixture further contains a chimeric oligonucleotide primer having a sequence substantially homologous to the nucleotide sequence of the nucleic acid as the template. A chimeric oligonucleotide primer represented by general formula below can be preferably used:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

In the chimeric oligonucleotide primer, c may be 0, the nucleotide analog may be deoxyriboinosine nucleotide or deoxyribouracil nucleotide and the modified ribonucleotide may be ($\alpha$-S) ribonucleotide. It is preferable to use a chimeric oligonucleotide primer consisting of a nucleotide sequence of any one of SEQ ID NOS:13 to 16, 23 to 26 and 28 to 31. The method may further comprise detecting an amplified nucleic acid using the probe of the first to fifth aspect.

The ninth aspect of the present invention relates to a chimeric oligonucleotide primer for detecting a pathogenic microorganism represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

According to the ninth aspect, one in which c is 0 can be preferably used. A chimeric oligonucleotide primer wherein the nucleotide analog is deoxyriboinosine nucleotide or deoxyribouracil nucleotide and the modified ribonucleotide is ($\alpha$-S) ribonucleotide is preferable. Although it is not intended to limit the present invention, for example, a chimeric oligonucleotide primer for detecting a pathogenic microorganism which is represented by any one of SEQ ID NOS:13 to 16, 23 to 26 and 28 to 31 is preferable.

The tenth aspect of the present invention relates to a primer for amplifying IS 6110 gene from a Mycobacterium tuberculosis complex and/or a fragment thereof, which has the nucleotide sequence of SEQ ID NO:36 or 37 or a sequence partially overlapping with said sequence; a primer for amplifying cppB gene from a gonococcus and/or a fragment thereof, which has the nucleotide sequence of SEQ ID NO:27 or a sequence partially overlapping with said sequence; a primer for amplifying pLGV440 from a chlamydia and/or a fragment thereof, which has the nucleotide sequence of SEQ ID NO:22 or a sequence partially overlapping with said sequence; and a primer for amplifying the 5' untranslated region from HCV and/or a fragment thereof, which has a nucleotide sequence of any one of SEQ ID NOS:41 to 43 or a sequence partially overlapping with said sequence.

According to the tenth aspect, the primer may be a chimeric oligonucleotide primer in which a part of the nucleotide sequence is replaced by a ribonucleotide. The primer of the ninth or tenth aspect may be labeled. The label is exemplified by a fluorescent substance, a dye, an enzyme, biotin or gold colloid.

The eleventh aspect of the present invention relates to a composition for detecting a target nucleic acid, which contains the probe of the first to fifth aspect.

The twelfth aspect of the present invention relates to a composition for detecting a target nucleic acid, which contains the primer of the ninth or tenth aspect.

The eleventh or twelfth aspect can be used for detecting a pathogenic, microorganism.

The thirteenth aspect of the present invention relates to a composition for detecting a pathogenic microorganism, which is used for the method for detecting a pathogenic microorganism of the sixth to eighth aspect, and which contains at least one reagent for amplification or hybridization of a target nucleic acid.

According to the thirteenth aspect, a reagent selected from the group consisting of a DNA polymerase having a strand displacement activity, an RNase H and a deoxyribonucleotide triphosphate may be contained. The DNA polymerase may be Bca DNA polymerase lacking 5'→3' exonuclease from Bacillus caldotenax, and the RNase H may be a type II RNase H from a bacterium belonging to genus Pyrococcus and/or a bacterium belonging to genus Archaeoglobus.

The fourteenth aspect of the present invention relates to a kit for detecting a pathogenic microorganism, which contains the probe of the first to fifth aspect.

According to the fourteenth aspect, the primer of the ninth or tenth aspect may be contained. The kit can be used to detect a Mycobacterium tuberculosis complex, a gonococcus, a chlamydia or HCV. The kit may be a kit which is used for the method for detecting a pathogenic microorganism of the sixth to eighth aspect, and which contains at least one reagent for amplification or hybridization of a target nucleic acid. A reagent selected from the group consisting of a DNA polymerase having a strand displacement activity, an RNase H and a deoxyribonucleotide triphosphate may be contained. The DNA polymerase is preferably Bca DNA polymerase lacking 5'→3' exonuclease from Bacillus caldotenax, and the RNase H may be a type II RNase H from a bacterium belonging to genus Pyrococcus and/or a bacterium belonging to genus Archaeoglobus. The kit may contain a support for capturing an amplification product. One in which the support is selected from the group consisting of a microtiter plate, a bead, a magnetic bead, a membrane and glass can be preferably used.

The 'fifteenth aspect of the present invention relates to a method for detecting a Mycobacterium tuberculosis complex, the method comprising treating a test sample containing a Mycobacterium tuberculosis complex with muramidase to extract a nucleic acid.

Brief Description of Drawings

Figure 1: a figure illustrating the relationship between the HCV-RNA concentration and the change in fluorescence intensity as well as the comparison of the difference in measurement range from the conventional method when measurements were carried out at varying HCV-RNA concentrations according to the method of the present invention.

Detailed Description of the Invention

As used herein, a deoxyribonucleotide (also referred to as a dN) refers to a nucleotide of which the sugar portion is composed of D-2-deoxyribose. The deoxyribonucleotides include, for example, ones having adenine, cytosine, guanine or thymine as the base portion. Furthermore, the deoxyribonucleotides also include a deoxyribonucleotide having a modified base such as 7-deazaguanosine and a deoxyribonucleotide analog such as deoxyinosine nucleotide.
As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which the oxygen atom of the phosphate group at the $\alpha$-position is replaced by a sulfur atom (also referred to as an ($\alpha$-S) ribonucleotide or an ($\alpha$-S) N) or other derivatives.
As used herein, a chimeric oligonucleotide primer refers to a primer that contains a deoxyribonucleotide and a ribonucleotide. The primer may contain a nucleotide analog and/or a modified ribonucleotide.
The chimeric oligonucleotide primers used in the present invention include any chimeric oligonucleotide primer that has a ribonucleotide being positioned at the 3'-terminus or on the 3'-terminal side of the primer, can be used to extend a nucleic acid strand in the method of the present invention, can be cleaved,with an endonuclease, and can be used to effect a strand displacement reaction.
As used herein, 3'-terminal side refers to a portion from the center to the 3'-terminus of a nucleic acid such as a primer. Likewise, 5'-terminal side refers to a portion from the center to the 5'-terminus of a nucleic acid.
As used herein, an endonuclease may be any one that acts on a double-stranded DNA generated by extending a DNA from the chimeric oligonucleotide primer which have been annealed to a nucleic acid as a template, and specifically cleaves it at a portion of the primer that contains a ribonucleotide.
As used herein, a DNA polymerase refers to an enzyme that synthesizes a DNA strand de novo using a DNA strand as a template. The DNA polymerases include naturally occurring DNA polymerases and variant enzymes having the above-mentioned activity. For example, such enzymes include a DNA polymerase having a strand displacement activity, a DNA polymerase lacking a 5' $\rightarrow$3' exonuclease activity and a DNA polymerase also having a reverse transcriptase activity or an endonuclease activity.
As used herein, "a strand displacement activity" refers to an activity that can effect a strand displacement, that is, that can proceed DNA duplication on the basis of the sequence of the nucleic acid as the template while displacing the DNA strand to release the complementary strand that has been annealed to the template strand. In addition, a DNA strand released from a nucleic acid as a template as a result of a strand displacement is referred to as "a displaced strand" herein.
As used herein, alkaline pH refers to pH above 7.
As used herein, a pathogenic microorganism refers to a pathogenic bacterium or virus.
As used herein, a target nucleic acid refers to any region of a gene derived from a pathogenic microorganism to be subjected to nucleic acid amplification or detection, which may be a DNA or an RNA.
Hereinafter, the present invention will be described in detail.

(1) Probe of the present invention

The probe of the present invention is characterized in that it can be used to detect a pathogenic microorganism such as a Mycobacterium tuberculosis complex, a gonococcus, a chlamydia or HCV. A probe that can stably hybridize to a target nucleic acid at alkaline pH exemplifies a preferred embodiment of the probe of the present invention. Although it is not intended to limit the present invention, for example, a probe that can hybridize to a target nucleic acid having a sequence complementary to the nucleotide sequence of the probe under alkaline conditions, for example at alkaline pH above 7.0, preferably within a range of pH 8-14, more preferably within a range of pH 8-10 exemplifies the probe that can hybridize at alkaline pH according to the present invention. Although it is not intended to limit the present invention, examples thereof include one containing the nucleotide sequence of SEQ ID NO:11, 20, 21, 34 or 35. The probe of the present invention may be used for hybridization under conventional conditions at neutral pH.
Using the probe of the present invention, a double-stranded nucleic acid denatured into single strands by alkali-treatment can be used in a hybridization step without neutralization. As a result, the procedure is simplified, and the sensitivity is increased by 10 times or more as compared with that of the conventional method. Furthermore, hybridization at alkaline pH can increase the hybridization specificity.

There is no specific limitation concerning the method for detecting a probe hybridized to a nucleic acid from a test sample. Any known detection method can be applied to the present invention.

By using the probe of the present invention labeled by a suitable means, a target nucleic acid in a sample can be detected efficiently and/or with a high sensitivity.

There is no specific limitation concerning the method for labeling a probe. For example, radioisotopes ($^{32}P$, etc.), dyes, fluorescent substances, luminescent substances, various ligands (biotin, digoxigenin, etc.), enzymes and the like can be used. The existence of the labeled probe can be confirmed using a detection method suitable for the label. In case of a ligand that cannot be detected directly, a substance that has a detectable label and that is capable of binding to the ligand may be used in combination. For example, a target nucleic acid can be detected with a high sensitivity by amplifying a signal using a ligand-labeled probe and an enzyme-labeled anti-ligand antibody in combination.

The probe may have a labeling substance for detection. For example, a ligand or receptor substance, a radioisotope, a fluorescent substance, a luminescent substance or a coloring substance can be preferably used as a labeling substance, without limitation.

A so-called homogeneous detection method can be preferably used according to the method of the present invention. In the homogeneous detection method, a probe having a labeling substance is hybridized to a nucleic acid obtained by amplifying the region of interest, and then detection is carried out after washing the free probe. Alternatively, the washing step may be omitted. For example, the fluorescence resonance energy transfer (FRET) method as described in Proc. Natl. Acad. Sci. USA, vol.85, p8790-8794 (1988), the molecular beacons method as described in Nature Biotechnology, vol.14, p303-308 (1996), the smart probe method as described in Anal. Chem., vol.72, p3717-3724 (2000) or the like can be preferably used as a homogeneous detection method utilizing fluorescence.

The molecular beacons method or the smart probe method in which the probe is labeled at the 5' end with a rhodamine-type dye or an oxazine-type dye, and at the 3' end with an oligonucleotide having a nucleotide sequence that causes quenching or a reporter dye, and in which the 5'-terminal side and the 3'-termiinal side of the probe are self-annealed exemplifies one embodiment of the present invention.

If such a probe is used in presence of a nucleic acid amplified using the amplification method of the present invention, the probe hybridizes to the amplified nucleic acid to cancel the self-annealed structure of the probe, resulting in emission of a fluorescent signal without suppression of the fluorescence intensity. On the other hand, if the amplified nucleic acid is absent, no fluorescent signal is detected because the probe keeps the self-annealed structure and the fluorescence intensity is suppressed.

Specifically, in case of the smart probe method, the fluorescent dye is preferably a rhodamine-type dye or an oxazine-type dye. The dye may be any one that is attached to the 5' end of the probe to cooperates with the nucleotide sequence at the 3' end. As a result of the cooperation, the fluorescence intensity is suppressed if the fluorescent probe is not attached to the target nucleic acid, whereas the fluorescent intensity is not suppressed if the fluorescent probe is attached to the target nucleic acid.

In case of the molecular beacons method, a combination in which the fluorescence intensity is suppressed due to fluorescence resonance energy transfer if the fluorescent probe is not attached to the target nucleic acid, whereas the fluorescence intensity is not suppressed when attached may be used.

FAM (6-carboxy-fluorescein), TAMRA (6-carboxy-tetramethyl-rhodamine), JA242 (oxazine) or DABCYL (4-dimethylaminoazobenzene-4'-sulfonyl chloride) is preferably used as the fluorescent dye. Such a fluorescent dye is known in the art and commercially available.

For example, a method as described in Nuc. Acids Res., vol.12, p3387-3403 (1984), J. Am. Chem. Soc., vol. 112, p1253-1254 (1990) or Anal. Chem., vol.70, p4771-4779 (1998) can be utilized as a method for fluorescence-labeling an oligonucleotide.

There is no specific limitation concerning the length of the probe of the present invention. The length is, for example 12-mer or more, preferably 20-mer or more, more preferably 40-mer or more.

The probe for detecting a Mycobacterium tuberculosis complex of the present invention can be used to detect Mycobacterium tuberculosis, Mycobacterium bovis BCG, Mycobacterium africanum, Mycobacterium microti and/or Mycobacterium canetti. The target nucleic acid for the probe is suitably selected depending on the nucleic acid to be detected or the organism. Although it is not intended to limit the present invention, for example, a probe for IS 6110 gene as a target nucleic acid can be used for detecting a Mycobacterium tuberculosis complex. Such a probe can be designed by selecting a suitable region from the nucleotide sequence of IS 6110 gene from a Mycobacterium tuberculosis complex represented by SEQ ID NO:12. Although it is not intended to limit the present invention, the sequence can be selected, for example from a region containing the nucleotide sequence of SEQ ID NO:39, preferably from a region containing the nucleotide sequence of SEQ ID NO:40, more preferably from a region containing the nucleotide sequence of SEQ ID NO:38. Particularly, the sequence can be selected from a region containing the nucleotide sequence of SEQ ID NO:11. Since such a probe hybridizes to IS 6110 gene from a Mycobacterium tuberculosis complex or a fragment thereof stably and with high specificity, it is particularly useful for detection of the gene and detection of a Mycobacterium tuberculosis complex.

The probe for detecting a gonococcus of the present invention can be used to detect Neisseria gonorrhoeae. The target nucleic acid for the probe is suitably selected depending on the nucleic acid to be detected or the organism. Although it is not intended to limit the present invention, for example, a probe for cryptic plasmid protein B (cppB) gene as a target nucleic acid can be used for detecting a gonococcus. Such a probe can be designed by selecting a suitable region from the nucleotide sequence of cppB gene from a gonococcus represented by SEQ ID NO:27. Although it is not intended to limit the present invention, the sequence can be selected, for example from a region containing the nucleotide sequence of SEQ ID NO:27, preferably from a region containing the nucleotide sequence of SEQ ID NO: 21. Since such a probe hybridizes to cppB gene from a gonococcus or a fragment thereof stably and with high specificity, it is particularly useful for detection of the gene and detection of a gonococcus.

The probe for detecting a chlamydia of the present invention can be used to detect Chlamydia trachomatis. The target nucleic acid for the probe is suitably selected depending on the nucleic acid to be detected or the organism. Although it is not intended to limit the present invention, for example, a probe for cryptic plasmid pLGV440 as a target nucleic acid can be used for detecting a chlamydia. Such a probe can be designed by selecting a suitable region from the nucleotide sequence of pLGV440 from a chlamydia represented by SEQ ID NO:22. Although it is not intended to limit the present invention, the sequence can be selected, for example from a region containing the nucleotide sequence of SEQ ID NO:22, preferably from a region containing the nucleotide sequence of SEQ ID NO:44, more preferably from a region containing the nucleotide sequence of SEQ ID NO:20. Since such a probe hybridizes to pLGV440 from a chlamydia or a fragment thereof stably and with high specificity, it is particularly useful for detection of the gene and detection of a chlamydia.

There is no specific limitation concerning the probe for detecting HCV of the present invention as long as it is a probe that can hybridize to a nucleotide sequence of a region bounded by a forward primer and a reverse primer to be used for amplification of a target nucleic acid. Although it is not intended to limit the present invention, the length of the probe is selected preferably within a range of 8 bases to 53 bases, more preferably within a range of 8 bases to 36 bases. For example, a probe having a nucleotide sequence of continuous 8 or more nucleotides within the above-mentioned range may be selected from the nucleotide sequence of SEQ ID NO:43. Although it is not intended to limit the present invention, a probe having a nucleotide sequence of continuous 8 or more nucleotides selected from the nucleotide sequence of SEQ ID NO:34 or 35 exemplifies such a probe.

(2) Detection method of the present invention

According to the method for detecting a target nucleic acid of the present invention, a step of neutralization following a step of denaturing a double-stranded target nucleic acid into single strands can be omitted by using the probe as described in (1) above. In other words, hybridization with a nucleic acid having a sequence complementary to the nucleotide sequence of the probe can be carried out under alkaline conditions at pH above 7, preferably at pH within a range of pH 8-14 according to the method for detecting a target nucleic acid of the present invention. The hybridization conditions include, but are not limited to, conditions known to those skilled in the art as "stringent conditions." Those as described in T. Maniatis et al. (eds.), Molecular Cloning: A Laboratory Manual 2nd ed., 1989, Cold Spring Harbor Laboratory or those as described in Examples herein can be used as such conditions.

Examples of typical compositions of hybridization solutions include the following: 6 x SSC (1 x SSC: 0.15 M NaCl, 0.015 M sodium citrate) containing 0.5% SDS, 5 x Denhardt's (0.1% bovine serum albumin (BSA), 0.1% polyvinylpyrrolidone, 0.1% Ficoll 400) and 100 μg/ml salmon sperm DNA. The pH of the hybridization solution may be adjusted to 8-14 according to the present invention.

Although it is not intended to limit the present invention, hybridization is carried out, for example, at a temperature lower than the Tm value of the probe by 5°C or more.

The Tm value of a probe can be determined, for example, according to the following equation:

$$Tm = 81.5 - 16.6(\log_{10}[Na^+]) + 0.41(\%G+C) - (600/N)$$

wherein N is the chain length of the probe; %G+C is the content of guanine and cytosine residues in the probe.

If the chain length of the probe is shorter than 18 bases, the Tm value can be estimated according to the following equation:

$$Tm = [(\%A+T) \times 2 + (\%G+C) \times 4]$$

wherein (%A+T): content of adenine and thymine residues in the probe; (%G+C): content of guanine and cytosine residues in the probe.

A probe suitable for detection of a target nucleic acid can be selected by confirming hybridization of the probe to the target nucleic acid under the above-mentioned hybridization conditions.

There is no specific limitation concerning the hybridization conditions according to the detection method of the present invention. Known hybridization conditions, preferably stringent hybridization conditions are used. The pH of the hybridization solution may be adjusted within a range of 8-14.

In a method for detecting a Mycobacterium tuberculosis complex as an exemplary embodiment of the method for detecting a target nucleic acid of the present invention, a probe suitable for detection of IS 6110 gene from a Mycobacterium tuberculosis complex can be preferably used. Although it is not intended to limit the present invention; a probe containing, for example the nucleotide sequence of SEQ ID NO:39 or a part thereof, preferably the nucleotide sequence of SEQ ID NO:40 or a part thereof, more preferably the nucleotide sequence of SEQ ID NO:38 or a part thereof, most preferably the nucleotide sequence of SEQ ID NO:11 can be preferably used according to the present invention. In a method for detecting a gonococcus, for example, a probe suitable for detection of cppB gene from a gonococcus can be preferably used. Although it is not intended to limit the present invention, a probe containing, for example the nucleotide sequence of SEQ ID NO:27 or a part thereof, preferably the nucleotide sequence of SEQ ID NO:21 or a part thereof can be preferably used according to the present invention. In a method for detecting a chlamydia, a probe suitable for detection of pLGV440 from a chlamydia can be preferably used. Although it is not intended to limit the present invention, a probe containing, for example the nucleotide sequence of SEQ ID NO:22 or a part thereof, preferably the nucleotide sequence of SEQ ID NO:44 or a part thereof, more preferably the nucleotide sequence of SEQ ID NO:20 or a part thereof can be preferably used according to the present invention. In a method for detecting HCV, the 5' untranslated region is preferable although it is not intended to limit the present invention. A probe containing, for example the nucleotide sequence of SEQ ID NO:43 or a part thereof, preferably the nucleotide sequence of SEQ ID NO:34 or a part thereof, or the nucleotide sequence of SEQ ID NO:35 or a part thereof can be preferably used according to the present invention.

According to the method for detecting a pathogenic microorganism of the present invention, respective pathogenic microorganisms can be specifically detected by using the probe as described in (1) above. Since the probe hybridizes to a gene from each pathogenic microorganism or a fragment thereof stably with high specificity, it is particularly useful for detection of the gene and detection of each pathogenic microorganism.

By using the probe as described in (1) above, hybridization between the probe and a sample containing a nucleic acid can be carried out under alkaline conditions at pH above 7, preferably at pH 8-14, more preferably at pH 8-10 according to the detection method of the present invention. Thus, the method provides a method for detecting a gene from a pathogenic microorganism or a fragment thereof which does not comprise a step of neutralization following a step of denaturation of a target nucleic acid. A Mycobacterium tuberculosis complex or the like contained in a test sample can be detected according to the method.

The method of the present invention can be utilized for detection or quantification of a specific gene in a sample. In other words, a specific gene can be detected or quantified from any sample that may contain a nucleic acid (DNA or RNA). Examples of the samples from which a specific gene can be detected or quantified include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples suspected to be contaminated or infected with a microorganism such as a virus or a bacterium (e.g., a food or a biological formulation), and samples that may contain an organism such as a soil and a waste water. Furthermore, the method can be utilized for detection of the presence of or quantification of the pathogenic microorganism based on the presence of the target nucleic acid, for example. Particularly, the method for detecting a pathogenic microorganism is useful in a field of hygiene or environment. An RNA or a DNA may be preferably used as a nucleic acid to be used as a template for the above-mentioned detection method.

A preparation containing a nucleic acid can be prepared from the above-mentioned material by using, for example, lysis with a detergent, sonication, shaking/stirring using glass beads or a French press, without limitation. In some cases, it is advantageous to further-process the preparation to purify the nucleic acid (e.g., in case where an endogenous nuclease exists). In such cases, the nucleic acid is purified by a know method such as phenol extraction, chromatography, ion exchange, gel electrophoresis or density-gradient centrifugation.

When a nucleic acid having a sequence derived from an RNA is use as a target, the method of the present invention may be conducted using as a template a cDNA synthesized by a reverse transcription reaction that uses the RNA as a template.

Any primer that anneals to an RNA as a template under reaction conditions used can be used in the reverse transcription reaction. The primer may be a primer having a nucleotide sequence that is complementary to a specific RNA as a template (a specific primer), an oligo-dT (deoxythymine) primer and a primer having a random sequence (a random primer). In view of specific annealing, the length of the primer for reverse transcription is preferably 6 nucleotides or more, more preferably 9 nucleotides or more. In view of oligonucleotide synthesis, the length is preferably 100 nucleotides or less, more preferably 30 nucleotides or less. A chimeric oligonucleotide primer can be used as a primer for reverse transcription. The chimeric oligonucleotide primer can also be utilized as a primer for a strand displacement reaction in the method for amplifying a nucleic acid of the present invention using a cDNA obtained after reverse transcription as a template. Such a primer may be any one that has the properties as described in (3) below and that can be used in a reverse transcription reaction from an RNA. For example, a primer having the nucleotide sequence of SEQ ID NO:32 or 33 can be preferably used.

Any enzyme that has an activity of synthesizing a cDNA using an RNA as a template can be used in the reverse transcription reaction. Examples thereof include reverse transcriptases originating from various sources such as avian myeloblastosis virus-derived reverse transcriptase (AMV RTase), Molony murine leukemia virus-derived reverse transcriptase (MMLV RTase) and Rous-associated virus 2 reverse transcriptase (RAV-2 RTase). In addition, a DNA polymerase that also has a reverse transcription activity can be used. An enzyme having a reverse transcription activity at a high temperature such as a DNA polymerase from a bacterium of genus Thermus (e.g., Tth DNA polymerase) and a DNA polymerase from a thermophilic bacterium of genus Bacillus is preferable for the present invention. For example, DNA polymerases from thermophilic bacteria of genus Bacillus such as a DNA polymerase from B. st (Bst DNA polymerase) and Bca DNA polymerase are preferable, although it is not intended to limit the present invention. For example, Bca DNA polymerase does not require a manganese ion for the reverse transcription reaction. Furthermore, it can synthesize a cDNA while suppressing the formation of a secondary structure of an RNA as a template under high-temperature conditions. Both a naturally occurring one and a variant of the enzyme having a reverse transcriptase activity can be used as long as they have the activity.

Both of a double-stranded DNA such as a genomic DNA isolated as described above or a PCR fragment and a single-stranded DNA such as a cDNA prepared by a reverse transcription reaction from a total RNA or an mRNA can be preferably used as a template DNA in the nucleic acid amplification method used in the present invention. The double-stranded DNA is preferably used after denaturing it into single-stranded DNAs.

As described above, a region on a target nucleic acid that hybridizes with the probe of the present invention can be used for hybridization after it is amplified using a suitable nucleic acid amplification method. There is no specific limitation concerning the nucleic acid amplification method to be used as long as it can be used to amplify a region on a target nucleic acid. For example, nucleic acid amplification methods that can be used include: the polymerase chain reaction (PCR) method (United States Patent Nos. 4,683,195, 4,683,202 and 4,800,159); the ligase chain reaction (LCR); the strand displacement amplification (SDA) method (JP-B 7-114718); the self-sustained sequence replication (3SR) method; the nucleic acid sequence based amplification (NASBA) method (Japanese Patent No. 2650159); the transcription-mediated amplification (TMA) method; the Qβ replicase method (Japanese Patent No. 2710159); and the isothermal and chimeric primer-initiated amplification of nucleic acids (ICAN) (WO 00/56877).

The ICAN method is a method in which a DNA having a specific nucleotide sequence on a temperate nucleic acid is successively amplified under isothermal conditions using a chimeric oligonucleotide primer, an endonuclease and a DNA polymerase having a strand displacement activity.

"Successively" means that a reaction proceeds without a change in the reaction temperature or the composition of the reaction mixture. As used herein, "isothermal" means conditions of a substantially constant temperature under which an enzyme and a nucleic acid strand function in each step.

Usually, one composed of deoxyribonucleotide(s) and ribonucleotide(s) and having the ribonucleotide(s) being positioned at the 3'-terminus or on the 3'-terminal side is used as a chimeric oligonucleotide primer. For example; an oligonucleotide having a structure represented by general formula below can be used as a primer for the ICAN method:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

For example, deoxyriboinosine nucleotide can be used as a nucleotide analog, and ($\alpha$-S) ribonucleotide can be used as a modified ribonucleotide. The nucleotide analog can be contained as long as the incorporation does not substantially abolish the function as a primer.

Based on the nucleotide sequences 5' and 3' to the region on the template of which the amplification is desired, two primers (one having a sequence homologous to the 5' nucleotide sequence and another complementary to the 3' nucleotide sequence) are prepared and used for amplification.

Any endonuclease that can act on a double-stranded DNA generated by DNA extension from the above-mentioned chimeric oligonucleotide primer that has been annealed to a nucleic acid as a template and cleaves the extended strand to effect a strand displacement reaction may be used in the present invention. That is, the endonuclease is an enzyme that can generate a nick in the chimeric oligonucleotide primer portion of the double-stranded DNA. Examples of endonucleases that can be used in the present invention include, but are not limited to, ribonucleases. In particular, endoribonuclease H (RNase H) that acts on an RNA portion of a double-stranded nucleic acid composed of a DNA and an RNA can be preferably used. Any ribonuclease that has the above-mentioned activities can be preferably used in the present invention, including mesophilic and heat-resistant ones. For example, an RNase H from E. coli can be used for a reaction at about 50°C to about 70°C in the method of the present invention as described below in Examples. A heat-resistant ribonuclease can be also preferably used in the method of the present invention. Examples of the heat-resistant ribonucleases which can be preferably used include, but are not limited to, a commercially available ribonuclease, Hybridase™ Thermostable RNase H (Epicenter Technologies) as well as an RNase H from a thermophilic bacterium of genus Bacillus, a bacterium of genus Thermus, a bacterium of genus Pyrococcus, a bacterium of genus Thermotoga, a bacterium of genus Archaeoglobus, a bacterium of genus Methanococcus or the like. Furthermore, both of naturally occurring ribonucleases and variants can be preferably used. The enzymatic unit of RNase H indicated herein is a value expressed according to a method of measuring an enzymatic unit as described in Referential Examples.

The RNase H is not limited to a specific one as long as it can be used in the method of the present invention. For example, the RNase H may be derived from various viruses, phages, prokaryotes or eukaryotes. It may be either a cellular RNase H or a viral RNase H. The cellular RNase H is exemplified by Escherichia coli RNase HI and the viral RNase H is exemplified by RNase H from HIV-1. Type I, type II or type III RNase H can be used in the method of the present invention. For example, RNase HI from Escherichia coli, or RNase HII from a bacterium of genus Pyrococcus or a bacterium of genus Archaeoglobus can be preferably used, without limitation.

The efficiency of the cleavage reaction with an endonuclease such as RNase H used in the method of the present invention may vary depending on the nucleotide sequence around the 3'-terminus of the primer and influence the amplification efficiency of the desired DNA. Therefore, it is natural to design the optimal primer for the RNase H used.

A DNA polymerase having a strand displacement activity on a DNA is used in the ICAN method. Particularly, a DNA polymerase having substantially no 5'→3' exonuclease activity can be preferably used.

Any DNA polymerases having the strand displacement activity can be used in the ICAN method without limitation. Examples thereof include variants of DNA polymerases lacking their 5'→3' exonuclease activities derived from thermophilic bacteria of genus Bacillus such as Bacillus caldotenax (hereinafter referred to as B. ca) and Bacillus stearothermophilus (hereinafter referred to as B. st), as well as large fragment (Klenow fragment) of DNA polymerase I from Escherichia coli. Both of mesophilic and heat-resistant DNA polymerases can be preferably used in the present invention. The enzyme may be either an enzyme purified from its original source or a recombinant protein produced by using genetic engineering techniques. The enzyme may be subjected to modification such as substitution, deletion, addition or insertion by using genetic engineering techniques or other means. Examples of such enzymes include BcaBEST DNA polymerase (Takara Shuzo), which is Bca DNA polymerase lacking its 5'→3' exonuclease activity. This enzyme exhibits its activity at 50°C to 70°C and can be preferably used for the method.

It is known that some DNA polymerases have an endonuclease activity such as an RNase H activity under specific conditions. Such a DNA polymerase can be used in the method of the present invention. In one aspect, the DNA polymerase may be used under conditions that allow the RNase H activity to express, e.g., in the presence of $Mn^{2+}$. In this case, the method of the present invention can be conducted without the addition of an RNase H. The aspect in which the Bca DNA polymerase can exhibit an RNase activity in a buffer containing $Mn^{2+}$ is not limited to the use of the Bca DNA polymerase. DNA polymerases that are known to have an RNase H activity such as Tth DNA polymerase from Thermus thermophilus can be used in the present invention.

The ICAN method is carried out by mixing a chimeric oligonucleotide primer, a sample containing a nucleic acid as a template, an endonuclease, a DNA polymerase, a deoxyribonucleotide triphosphate (dNTP) and the like in a buffer having a composition in which the enzymes can exhibit their activities, and incubating the reaction mixture at an appropriate temperature for a sufficient time to generate a reaction product.

Prior to the reaction, a chimeric oligonucleotide primer and a nucleic acid as a template may be mixed, incubated at a temperature at which a double-stranded nucleic acid is denatured (e.g., 90°C or above), and cooled to the reaction temperature used for the method of the present invention or below for annealing, although such steps are not indispensable to the amplification reaction.

If an RNA is used as a template in the detection method of the present invention, a reverse transcription reaction and a nucleic acid amplification reaction may be carried out in one step. Although it is not intended to limit the present invention, for example, a combination of AMV RTase, MMLV RTase or RAV-2 RTase with Bca DNA polymerase can be preferably used as a combination of a reverse transcriptase and a strand displacement-type DNA polymerase. Alternatively, one DNA polymerase having a reverse transcriptase activity and a strand displacement activity may be used.

The method for detecting a target nucleic acid of the present invention can be conducted by amplifying the target nucleic acid directly from a sample containing the nucleic acid. In this case, the chain length of the target nucleic acid to be amplified is not limited to a specific one. For example, a region of 200 bp or shorter, preferably 150 bp or shorter is effective for sensitive detection of the target nucleic acid. The target nucleic acid in the sample can be detected with a high sensitivity by designing the chimeric oligonucleotide primers of the present invention to result in the chain length to be amplified as described above.

In addition, a target nucleic acid can be detected with a higher sensitivity even from a trace amount of a nucleic acid sample in the detection method of the present invention by using a reaction buffer containing Bicine, Tricine, HEPES, phosphate or tris as a buffering component and an annealing solution containing spermidine or propylenediamine. In this case, the endonuclease and the DNA polymerase to be used are not limited to specific ones. For example, a combination of an RNase H from Escherichia coli, a bacterium of genus Pyrococcus or a bacterium of genus Archaeoglobus and BcaBEST DNA polymerase is preferable. It is considered that the preferable units of the endonuclease and the DNA polymerase may vary depending on the types the enzymes. In such a case, the composition of the buffer and the amount of the enzymes added may be adjusted using the increase in the detection sensitivity or the amount of the amplification product as an index.

In the aspect of the nucleic acid amplification method in which a double-stranded DNA as a template and two chimeric oligonucleotide primers are used, switching of templates may occur among the template-extended strand intermediates during the extension reaction from the primers to generate a double-stranded nucleic acid consisting of the synthesized primer-extended strands being annealed each other, although it depends on the reaction conditions or the like. The double-stranded nucleic acid has chimeric oligonucleotide primers at both ends. Then, reaction of extending complementary strands comprising strand displacement can be initiated from both of the ends again. As a result of the reaction, an amplification product having the primer sequence at one end is generated. Furthermore, if switching of templates occurs during the reaction, a double-stranded nucleic acid similar to one that described above is generated again.

A method for amplifying a nucleic acid comprising conducting a template switching reaction using a DNA polymerase having a strand displacement activity as described above can be utilized according to the present invention.

For example, a variant enzyme of Bca DNA polymerase lacking a 5'→3' exonuclease activity is preferably used as a DNA polymerase capable of effecting the template switching reaction during strand displacement reaction in particular. Such an enzyme is commercially available as BcaBEST DNA polymerase (Takara Shuzo). It can also be prepared from Escherichia coli HB101/pU1205 (FERM BP-3720) which contains the gene for the enzyme according to the method as described in Japanese Patent No. 2978001. Escherichia coli HB101/pU1205 (FERM BP-3720) has been deposited at International Patent Organism Depositary, National Institute of Advanced Industrial Science and Technology, AIST Tsukuba Central 6, 1-1, Higashi 1-chome, Tsukuba-shi, Ibaraki 305-8566, Japan since May 10, 1991 (date of original deposit).

In the detection method of the present invention, dUTP may be incorporated as a substrate during amplification of a target nucleic acid. Thus, if dUTP is used as a substrate, it is possible to prevent false positive due to carry-over contamination of amplification products by degrading amplification products utilizing uracil N-glycosidase (UNG).

According to the detection method of the present invention, known methods for detecting a nucleic acid can be used for detecting a target nucleic acid amplified using the above-mentioned nucleic acid amplification method. Examples of such methods include detection of a reaction product having a specific size by electrophoresis, and detection by hybridization with a probe. Furthermore, a detection method in which magnetic beads or the like are used in combination can be preferably used. A fluorescent substance such as ethidium bromide is usually used in the detection by electrophoresis. The hybridization with a probe may be combined with the detection by electrophoresis. The probe may be labeled with a radioisotope or with a nonradioactive substance such as biotin or a fluorescent substance. Additionally, use of a labeled nucleotide in step (b) may facilitate the detection of amplification product into which the labeled nucleotide is incorporated, or may enhance the signal for detection utilizing the label. A fluorescence polarization method, a fluorescence energy transfer or the like can also be utilized for the detection. The target nucleic acid can be detected automatically or quantified by constructing a suitable detection system. In addition, detection with naked eyes by a hybrid chromatography method can be preferably used.

A ribonucleotide (RNA) probe, or a chimeric oligonucleotide probe composed of ribonucleotide(s) and deoxyribonucleotide(s), that is labeled with two or more fluorescent substances positioned at a distance that results in a quenching state can be used in the detection method of the present invention. The probe does not emit fluorescence. When it is annealed to a DNA amplified from a target nucleic acid that is complementary to the probe, RNase H digests the probe. The distance between the fluorescent substances on the probe then increases, resulting in the emission of fluorescence. Thus, the emission reveals the presence of the target nucleic acid. If RNase H is used in the method for amplifying a nucleic acid of the present invention, a target nucleic acid can be detected only by adding the probe to the reaction mixture. For example, a combination of fluorescent substances, 6-carboxyfluorescein (6-FAM) and N,N,N',N'-tetramethyl-6-carboxyrhodamine (TAMRA), can be preferably used for labeling the probe.

The isothermal amplification method used for the detection method of the present invention does not require the use of equipment such as a thermal cycler. The number of primers used in the amplification method of the present invention can be one or two, which is less than that used in a conventional method. Since reagents such as dNTPs used for PCR and the like can be applied to the method of the present invention, the running cost can be reduced as compared with a conventional method. Therefore, the method of the present invention can be preferably used, for example, in a field of genetic test in which the detection is routinely conducted. The method of the present invention provides a greater amount of an amplification product in a shorter time than the PCR. Therefore, the method of the present invention can be utilized as a convenient, rapid and sensitive method for detecting a gene.

When the detection method of the present invention is used, a means for making the reaction system small and increasing the degree of integration may be used in combination in order to analyze a large number of test samples. A system in which basic processes for the detection method of the present invention (e.g., extraction of DNA from cells, nucleic acid amplification reaction, detection of the DNA of interest, etc.) are integrated on a microchip of several square centimeters to fingertip size by utilizing the latest hyperfine processing techniques may be used in combination as such a means. Furthermore, a process of gel or capillary electrophoresis, or hybridization with a detection probe may be optionally included in combination. Such a system is called a microchip, micro CE (capillary electrophoresis) chip or a nanochip.

Any nucleic acid amplification reaction can be utilized for the system as long as the DNA fragment of interest is amplified using the method. Although it is not intended to limit the present invention, for example, a method that can be used to amplify a nucleic acid under isothermal conditions such as the ICAN method can be preferably used. Since the system can be simplified using such a method in combination, the method is utilized very suitably for the integrated system as described above. A more highly integrated system can be constructed by utilizing the techniques of the present invention.

Although it is not intended to limit the present invention, for example, the following chimeric oligonucleotide primer can be preferably used according to the detection method of the present invention: a chimeric oligonucleotide primer for detection of a Mycobacterium tuberculosis complex having a nucleotide sequence represented by any one of SEQ ID NOS:13 to 16; a chimeric oligonucleotide primer for detection of a gonococcus having a nucleotide sequence represented by SEQ ID NO:28 or 29; a chimeric oligonucleotide primer for detection of a chlamydia having a nucleotide sequence represented by any one of SEQ ID NOS:23 to 26; or a chimeric oligonucleotide primer for detection of HCV having a nucleotide sequence represented by SEQ ID NOS:30 or 31. The amplification product of interest can be confirmed by amplification of a target nucleic acid in a sample using such a chimeric oligonucleotide primer followed by electrophoresis or real-time detection.

Furthermore, a target nucleic acid can be detected using the following probes according to the detection method of the present invention: a probe for detection of a Mycobacterium tuberculosis complex selected from a region containing a nucleotide sequence represented by any one of SEQ ID NOS:11, 12 and 38 to 40; a probe for detection of a gonococcus selected from a region containing a nucleotide sequence represented by SEQ ID NO:27; a probe for detection of a chlamydia selected from a region containing a nucleotide sequence represented by SEQ ID NO:22 or 44; or a probe for detection of HCV selected from a region containing a nucleotide sequence represented by any one of SEQ ID NOS:43 to 45.

If an isothermal nucleic acid amplification method is used in the method of the present invention, only a thermostat bath is necessary as reaction equipment, and no strict equipment such as a thermal cycler is required. A commercially available spectrophotometer, fluorescence detector, plate reader or the like can be used as equipment for measuring fluorescent signals. Thus, it is possible to rapidly conduct measurements on a large number of test samples in a place for conventional examination works. Reagents necessary for the method of the present invention are also commercially available.

HCV is detected using the method of the present invention as follows: a mixture containing an RNA from HCV extracted from a test sample according to a conventional method, a primer for reverse transcription reaction, a reverse transcriptase (not necessary if a DNA polymerase having a reverse transcription activity is used) and dNTPs (including dATP, dGTP, dCTP and dTTP) is prepared; a target nucleic acid is amplified using a cDNA generated from the HCV-RNA as a template, the chimeric oligonucleotide primer and a DNA polymerase for ICAN at a constant temperature; hybridization of the amplification product is carried out with the fluorescence-labeled probe in a homogeneous system; and the fluorescence intensity is measured. Specific conditions for the reaction are described in detail in Examples below.

In the reaction, a cDNA is first synthesized from the HCV-RNA as a template, and DNA amplification takes place by the ICAN method using the cDNA as a template. Since the amplified DNA contains a region complementary to the fluorescent probe, the fluorescent probe hybridizes to the single-stranded amplified DNA. Hybridization of the fluorescent probe cancels the suppression of fluorescence intensity, resulting in increase in the fluorescence intensity. On the other hand, if a sample contains no RNA for HCV, hybridization does not take place, and the fluorescence intensity remains suppressed and is weak. Thus, the RNA for HCV in a sample can be detected conveniently, rapidly and with a high sensitivity by measuring the fluorescence intensity.

According to the method of the present invention, the RNA for HCV is detected based on the fluorescence intensity in a homogeneous system without a step of washing. The present invention is advantageous in that it can deal with simultaneous measurements of multiple items by changing the fluorescent dye and measurement wavelength. Specifically, HBV, HIV and HTLV, in addition to HCV, are also important measurement items in a blood center. The items can be simultaneously measured by using primers for ICAN and probes specific for the respective items and labeling the probes with fluorescent dyes with different measurement wavelengths. Measurements of the fluorescence intensities at the endpoints of reactions enable sensitive and qualitative measurements of a large number of test samples, for example, in a blood center. On the other hand, kinetic fluorescence measurements of reactions enable quantitative measurements for monitoring therapies. Thus, the method of the present invention is very convenient because it does not require a complicated and special instrument for adjusting the temperature up and down which is required for the conventional RT-PCR method, it improves inconvenience that a large number of test samples cannot be measured in a limited period and facilities, and it does not require a step of washing.

(3) Primer of the present invention

A chimeric oligonucleotide primer exemplifies a primer used for the detection method of the present invention. A primer composed of a deoxyribonucleotide, a nucleotide analog, or an unmodified or modified ribonucleotide with the ribonucleotide being positioned at the 3'-terminus or on the 3'-terminal side of the primer is used as the primer. For example, an oligonucleotide having a structure represented by general formula below can be used as a primer for the ICAN method:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

For example, deoxyriboinosine nucleotide, deoxyribouracil nucleotide, a modified deoxyribonucleotide or the like can be used as a nucleotide analog, and ($\alpha$-S) ribonucleotide can be used as a modified ribonucleotide.

Based on the nucleotide sequences 5' and 3' to the region on a template of which the amplification is desired, two primers (one having a sequence homologous to the 5' nucleotide sequence and another complementary to the 3' nucleotide sequence) are prepared and used for amplification.

For example, a Mycobacterium tuberculosis complex can be detected according to the present invention by carrying out a nucleic acid amplification reaction according to the above-mentioned method using a sample for which a Mycobacterium tuberculosis complex is to be detected and then carrying out hybridization between the amplification product and the probe of the present invention. A primer for nucleic acid amplification can be designed and prepared with reference to the nucleotide sequence of IS 6110 gene from a Mycobacterium tuberculosis complex as shown in SEQ ID NO:12 such that it is suitable for use in various nucleic acid amplification methods. Although it is not intended to limit the present invention, one that can be used to amplify the following region in IS 6110 gene is preferable for the present invention: for example, a region containing the nucleotide sequence of SEQ ID NO:39 or a part thereof; preferably, a region containing the nucleotide sequence of SEQ ID NO:40 or a part thereof; more preferably, a region containing the nucleotide sequence of SEQ ID NO:38 or a part thereof. In addition, one that can be used to amplify a region containing the nucleotide sequence of SEQ ID NO:11 is preferable.

Furthermore, a highly sensitive and quantitative nucleic acid amplification can be carried out using the primer of the present invention obtained by analyzing the polymorphism among the previously reported nucleotide sequences of Mycobacterium tuberculosis complex IS 6110 using the GenBank gene database, and conducting searches including related sequences homologous to Mycobacterium tuberculosis complex IS 6110, preferably, using a primer for nucleic acid amplification having the nucleotide sequence of SEQ ID NO:36 or 37. Such a primer is useful for amplification of IS 6110 gene from a Mycobacterium tuberculosis complex or a fragment thereof using various nucleic acid amplification methods.

Like the detection of a Mycobacterium tuberculosis complex as described above, a probe and a chimeric oligonucleotide primer can be suitably used to detect a gonococcus, a chlamydia or HCV according to the present invention.

The primer is not limited to one having the above-mentioned nucleotide sequence. One having a sequence around the nucleotide sequence (i.e., a sequence partially overlapping with the sequence) can be preferably used according to the present invention. A primer can be prepared by suitably selecting a nucleotide sequence overlapping with said sequence depending on the feature of the nucleic acid amplification method to be used.

The chimeric oligonucleotide primer can be synthesized to have desired nucleotide sequence using, for example, the 394 type DNA synthesizer from Applied Biosystems Inc. (ABI) according to a phosphoramidite method.

Alternatively, any methods including a phosphate triester method, an H-phosphonate method and a thiophosphonate method may be used to synthesize the chimeric oligonucleotide primer.

One in which deoxyribonucleotide(s) in the 3' end portion of the primer is (are) replaced by ribonucleotide (s) so that it can be used for the ICAN method may be used as a primer for nucleic acid amplification using the ICAN method. Examples of such primers include chimeric oligonucleotide primers each having the nucleotide sequence of any one of SEQ ID NOS:13-16, 23-26 and 28-31.

The primer may be labeled with a suitable substance such as a fluorescent substance, a dye, a ligand (biotin, digoxigenin, etc.) or gold colloid. For example, a sensitive, convenient and quantitative measurement method is provided by capturing, onto a support, a target nucleic acid amplified using a primer labeled with a ligand. In this case, a microtiter plate, a bead, a magnetic bead, a membrane and glass are exemplary solid phases.

A DNA sample extracted from a sputum test sample may contain a DNA derived from a respiratory tract-indigenous bacterium or a virus in addition to a human DNA. Then, amplification of a gene from a Mycobacterium tuberculosis complex was tested using as a test sample a sputum from a volunteer who had never infected with a Mycobacterium tuberculosis complex in order to examine the specificity of the primer developed according to the present invention. No positive result in such a test can demonstrate the specificity of the primer. Amplification of an IS 6110 gene fragment from each of the DNAs extracted from sputa collected from 38 volunteers was carried out by the ICAN method using the chimeric oligonucleotide primer. As a result, no positive result was observed, demonstrating that the specificity of the primer for a Mycobacterium tuberculosis complex is high and that no false positive result is obtained from a non-IS 6110 target DNA.

(4) Composition of the present invention

The present invention provides a composition used for the detection method of the present invention as described in (2) above. Examples of the compositions include one containing the probe as described in (1) above and the primer as described in (3) above. Alternatively, the composition may be in a form comprising a composition for detection containing the probe and a composition for amplification containing the primer. The composition may be in a form comprising a composition containing magnesium acetate and a composition containing the primer. The composition may contain a buffering component, dNTPs or the like. It may contain a modified deoxyribonucleotide or a deoxyribonucleotide triphosphate analog. Furthermore, it may contain an internal control (IC) for checking failure in amplification (false negative) of the reagent in the composition of the present invention due to degradation, inactivation or the like. The internal control can be amplified using the primer of he present invention.

A composition containing the above-mentioned various components at suitable concentrations for the detection method of the present invention exemplifies another embodiment. An amplification reaction can be carried out only by adding an appropriate template and an appropriate chimeric oligonucleotide primer to such a composition. If the subject of amplification is known in advance, a composition containing a chimeric oligonucleotide primer suitable for amplification of the subject is preferable.

A composition containing the above-mentioned various components at concentrations suitable for the nucleic acid amplification method of the present invention exemplifies a particularly preferable embodiment. An amplification reaction can be carried out only by adding an appropriate template to such a composition. If the composition contains a detection probe, a target nucleic acid derived from a pathogenic microorganism can be detected in a real-time manner.

Although it is not intended to limit the present invention, for example, by using, for detection of HCV, the method of the present invention, or a composition or a kit for the method, the measurement range can be made broader up to three orders of magnitude as compared with that of the conventional Amplicor HCV kit (two orders of magnitude). Furthermore, the total time can be greatly shortened to about 45 minutes according to the present invention as compared with the total time of about five hours required for the conventional Amplicor HCV kit. Accordingly, the number of test samples that can be handled in a day can be increased.

(5) Kit of the present invention

The kit of the present invention is a kit for detecting a target nucleic acid from a pathogenic microorganism. Although it is not intended to limit the present invention, it contains a probe that can hybridize to a target nucleic acid under alkaline conditions at pH above 7, for example, the probe as described in (1) above.

A kit for detecting a Mycobacterium tuberculosis complex exemplifies one embodiment of the kit of the present invention. The kit contains a probe that can be used to specifically detect Mycobacterium tuberculosis, Mycobacterium bovis BCG, Mycobacterium africanum, Mycobacterium microti and/or Mycobacterium canetti. The kit can be used to detect a Mycobacterium tuberculosis complex under alkaline conditions at pH above 7.

A kit for detecting a gonococcus exemplifies one embodiment of the kit of the present invention. The kit contains a probe that can be used to specifically detect Neisseria gonorrhoeae. The kit can be used to detect a gonococcus under alkaline conditions at pH above 7.

A kit for detecting a chlamydia exemplifies one embodiment of the kit of the present invention. The kit contains a probe that can be used to specifically detect Chlamydia trachomatis. The kit can be used to detect a chlamydia under alkaline conditions at pH above 7.

A kit for detecting HCV exemplifies one embodiment of the kit of the present invention. The kit contains a probe that can be used to specifically detect HCV. The kit can be used to detect HCV under alkaline conditions at pH above 7.

In another embodiment, the kit may contain a primer. The kit may contain a reagent for a nucleic acid amplification method for amplifying a target gene (e.g., a DNA polymerase), a reagent used for a detection reaction of a probe, a reagent used for extracting a nucleic acid from a test sample or the like. Such a kit is preferable for carrying out the method for detecting a Mycobacterium tuberculosis complex or the like of the present invention conveniently and rapidly. In particular, the kit can be used to provide test results with high reproducibility and reliability when existence of a Mycobacterium tuberculosis complex or the like is examined for a number of samples. Furthermore, the kit may contain an internal control (IC) for checking false negative and a probe for detecting the internal control. The internal control can be amplified using the primer of he present invention.

In one embodiment, the kit is in a packed form and contains instructions regarding the use of a probe, a primer, a DNA polymerase and an endonuclease according to the present invention. A commercially available DNA polymerase and/or endonuclease may be selected and used according to the instructions. Additionally, the kit may contain a reagent for a reverse transcription reaction that is used when an RNA is used as a template. The DNA polymerase can be selected from the DNA polymerases as described above. The endonuclease can be selected from Pfu RNase HII or Afu RNase HII.

"Instructions" are printed matters describing a method of using the kit, e.g., a method for preparing a reagent solution for a strand displacement reaction, recommended reaction conditions and the like. The instructions include an instruction manual in a form of a pamphlet or a leaflet, a label stuck to the kit, and description on the surface of the package containing the kit. The instructions also include information disclosed or provided through electronic media such as the Internet.

The kit of the present invention may further contain a reaction buffer containing Bicine, Tricine, HEPES, phosphate or tris as a buffering component and an annealing solution. Additionally, it may contain a DNA polymerase and an RNase H. Furthermore, the kit may contain a modified deoxyribonucleotide or a deoxynucleotide triphosphate analog.

By appropriately selecting a detection probe for the kit of the present invention, a kit that can be used to detect a target nucleic acid from a pathogenic microorganism in a real-time manner is provided.

(6) Nucleic acid extraction method of the present invention

The present invention provides a method for extracting a nucleic acid which is used for detecting a Mycobacterium tuberculosis complex or the like with a high sensitivity. Extraction of a nucleic acid from a clinical specimen is a delicate and troublesome procedure. The range of subjects for the procedure is wide, including liquid test samples (urine, pleural fluid, spinal fluid, blood, etc.), thick viscous test samples (pus, sputum, etc.), cells and tissues. Currently, there is no standard method for extracting a gene from a Mycobacterium tuberculosis complex or the like from such a test sample. Therefore, various nucleic acid extraction methods are used in reports concerning genetic tests for a Mycobacterium tuberculosis complex or the like to date. In the conventional methods, mixing in a solution containing a detergent, an alkali, an organic solvent or a chaotropic reagent, or sonication in the presence of glass beads are carried out.

The present inventors compared the above-mentioned various conventional methods with a method in which muramidase (one derived from an actinomycete Streptomyces globisporus is sold by Sigma under the name of mutanolysin), which has been used for extraction of nucleic acids from microorganisms belonging to genus Listeria, genus Lactobacillus and genus Streptococcus, was used for a Mycobacterium tuberculosis complex. Then, the present inventors have found that the method in which a Mycobacterium tuberculosis complex is digested with muramidase is the most excellent. Furthermore, the present inventors have found that more excellent results can be obtained by boiling the cells digested with the enzyme for five minutes.

Detection of a Mycobacterium tuberculosis complex by treatment of a sample containing a Mycobacterium tuberculosis complex with muramidase for 30 minutes followed by treatment at 96°C for five minutes and amplification of a gene using the ICAN method was examined. As a result, it was surprisingly found that a DNA from a Mycobacterium tuberculosis complex could be detected with a sensitivity 10-fold higher than the conventional method in which a reagent such as a detergent was used. This detection sensitivity corresponds to five copies of the Mycobacterium tuberculosis complex genome.

The above-mentioned sequential detection procedure can exclude the inhibitory effect, on a nucleic acid amplification reaction, of a component which may contaminate a nucleic acid extract according to a conventional method in which a detergent or a chaotropic reagent is used. Furthermore, the heating step provides an effect of sterilizing a Mycobacterium tuberculosis complex or the like. Thus, the method is advantageous in that it provides an effect of preventing infection with a Mycobacterium tuberculosis complex or the like in a laboratory which has been a trouble to a tester. Accordingly, a DNA can be extracted from a Mycobacterium tuberculosis complex or the like rapidly, safely and with a high sensitivity using the protocol of the present invention.

For example, if a sputum is used as a sample, it is preferable to first process the sputum according to the NALC (N-acetyl-L-cysteine)-NaOH method, which is a known method for processing a sputum, and then carry out the nucleic acid extraction method of the present invention as described above. The detection method of the present invention may comprise a step of treating a test sample containing a Mycobacterium tuberculosis complex or the like with muramidase to extract a nucleic acid.

The whole protocol used for the detection method of the present invention including the probe, the primer, the nucleic acid extraction method and the nucleic acid amplification method enables very rapid, reliable and highly sensitive detection of a Mycobacterium tuberculosis complex or the like. It is possible to complete a procedure from collection of a test sample to reporting of results within three hours using the method. The time required for a test is reduced by half as compared with the time (six hours) required for a conventional kit (for example, a kit from Roche). Thus, the method of the present invention enables provision of test results on the same day and immediate quarantine of the infected patient, and contributes much to the society in view of public health by early prevention of spread of tuberculosis.

The following Examples illustrate the present invention in more detail, but are not to be construed to limit the scope thereof. In addition, those skilled in the art can readily carry out by analogy based on the present invention an improvement in prevention of carry-over contamination of an amplification product, and an improvement in which an internal control, another Mycobacterium tuberculosis complex gene or the like are simultaneously amplified and detected.

## Examples

The following Examples illustrate the present invention in more detail, but are not to be construed to limit the scope thereof.

### Referential Example 1: Cloning of Pyrococcus furiosus RNase HII gene

#### (1) Preparation of genomic DNA from Pyrococcus furiosus

2 L of a medium containing 1% Tryptone (Difco Laboratories), 0.5% yeast extract (Difco Laboratories), 1% soluble starch (Nacalai Tesque), 3.5% Jamarine S Solid (Jamarine Laboratory), 0.5% Jamarine S Liquid (Jamarine Laboratory), 0.003% $MgSO_4$, 0.001% Nacl, 0.0001% $FeSO_4 \cdot 7H_2O$, 0.0001% $CoSO_4$, 0.0001% $CaCl_2 \cdot 7H_2O$, 0.0001% $ZnSO_4$, 0.1 ppm $CuSO_4 \cdot 5H_2O$, 0.1 ppm KA1 $(SO_4)_2$, 0.1 ppm $H_3BO_4$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Pyrococcus furiosus (purchased from Deutsche Sammlung von Mikroorganismen; DSM3638) was inoculated into the medium and cultured at 95°C for 16 hours without shaking. After cultivation, cells were collected by centrifugation.

The resulting cells were then suspended in 4 ml of 25% sucrose, 50 mM tris-HCl (pH 8.0). 0.4 ml of 10 mg/ml lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour.

After reaction, the mixture was subjected to phenol-chloroform extraction followed by ethanol precipitation to prepare about 1 mg of genomic DNA.

#### (2) Cloning of RNase HII gene

The entire genomic sequence of Pyrococcus horikoshii was published [DNA Research, 5:55-76 (1998)]. The existence of one gene encoding a homologue of RNase HII (PH1650) in the genome was known (SEQ ID NO:1, the home page of National Institute of Technology and Evaluation: http://www.nite.go.jp/).

Homology between the PH1650 gene (SEQ ID NO:1) and the partially published genomic sequence of Pyrococcus furiosus (the home page of University of Utah, Utah Genome Center: http://www.genome.utah.edu/sequence.html) was searched. As a result, a highly homologous sequence was found.

Primers 1650Nde (SEQ ID NO:2) and 1650Bam (SEQ ID NO:3) were synthesized on the basis of the homologous sequence.

A PCR was carried out in a volume of 100 µl using 200 ng of the Pyrococcus furiosus genomic DNA obtained in (1) as a template, and 20 pmol of 1650Nde and 20 pmol of 1650Bam as primers. TaKaRa Ex Taq (Takara Shuzo) was used as a DNA polymerase for the PCR according to the attached protocol. The PCR was carried out as follows: 30 cycles of 94°C for 30 seconds, 55°C for 30 seconds and 72°C for 1 minute. An amplified DNA fragment of about 0.7 kb was digested with NdeI and BamHI (both from Takara Shuzo). The resulting DNA fragment was inserted between the NdeI site and the BamHI site in a plasmid vector pET3a (Novagen) to make a plasmid pPFU220.

(3) Determination of nucleotide sequence of DNA fragment containing RNase HII gene

The nucleotide sequence of the DNA fragment inserted into pPFU220 obtained in (2) was determined according to a dideoxy method.

Analysis of the determined nucleotide sequence revealed the existence of an open reading frame (ORF) presumably encoding RNase HII. The nucleotide sequence of the open reading frame is shown in SEQ ID NO:4. The amino acid sequence of RNase HII deduced from the nucleotide sequence is shown in SEQ ID NO:5.

Escherichia coli JM109 transformed with the plasmid pPFU220 is designated and indicated as Escherichia coli JM109/pPFU220, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM P-18020 and transmitted to International Patent Organism Depositary, National Institute of Advanced Industrial Science and Technology under Budapest Treaty on July 9, 2001 (date of transmission to international depositary authority) under accession number FERM BP-7654.

(4) Preparation of purified RNase HII preparation

Escherichia coli HMS174(DE3) (Novagen) was transformed with pPFU220 obtained in (2). The resulting Escherichia coli HMS174(DE3) harboring pPFU220 was inoculated into 2 L of LB medium containing 100 µg/ml of ampicillin and cultured with shaking at 37°C for 16 hours. After cultivation, cells collected by centrifugation were suspended in 66.0 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 61.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

60.0 ml of the flow-through RNase HII fraction was subjected to RESOURSE S column (Amersham Pharmacia Biotech) equilibrated with Buffer A and eluted with a linear gradient of 0 to 500 mM NaCl using FPLC system. A fraction containing RNase HII eluted with about 150 mM NaCl was obtained.

2.0 ml of the RNase HII fraction was concentrated by ultrafiltration using Centricon-10 (Amicon). 250 µl of the concentrate was subjected to Superdex 200 gel filtration column (Amersham Pharmacia Biotech) equilibrated with 50 mM tris-HCl (pH 8.0) containing 100 mM NaCl and 0.1 mM EDTA and eluted with the same buffer. As a result, RNase HII was eluted at a position corresponding to a molecular weight of 17 kilodalton. This molecular weight corresponds to that of the RNase HII in the monomeric form.

The thus eluted RNase HII was used as Pfu RNase HII preparation. The RNase H activity of the thus obtained Pfu RNase HII preparation was measured as follows.

10 mM tris-HCl (pH 8.0), 1 mM dithiothreitol (Nacalai Tesque), 0.003% bovine serum albumin (fraction V, Sigma), 4% glycerol, 20 µg/ml poly(dT) (Amersham Pharmacia Biotech) and 30 µg/ml poly(rA) (Amersham Pharmacia Biotech) were mixed together. The mixture was incubated at 37°C for 10 minutes and used as a substrate solution for measuring an RNase H activity.

1 µl of 1 M $MnCl_2$ was added to 100 µl of the substrate solution. The mixture was incubated at 40°C. An appropriate dilution of the Pfu RNase HII preparation was added to the mixture to initiate a reaction. After reacting at 40°C for 30 minutes, 10 µl of 0.5 M EDTA was added thereto to terminate the reaction. Absorbance at 260 nm was then measured.

As a result, the absorbance at 260 nm observed for the reaction mixture to which the Pfu RNase HII preparation was added was higher than that observed for the reaction mixture to which the preparation was added following 10 µl of 0.5 M EDTA. Thus, it was demonstrated that the preparation has an RNase H activity.

Referential Example 2: Preparation of Afu RNase HII

Cloning of RNase HII gene from Archaeoglobus fulgidus

(1) Preparation of genomic DNA from Archaeoglobus fulgidus

Cells of Archaeoglobus fulgidus (purchased from Deutsche Sammlung von Mikroorganismen und Zellkulturen GmbH; DSM4139) collected from 8 ml of a culture were suspended in 100 µl of 25% sucrose, 50 mM tris-HCl (pH 8.0). 20 µl of 0.5 M EDTA and 10 µl of 10 mg/ml lysozyme chloride (Nacalai Tesque) in water were added thereto. The mixture was reacted at 20°C for 1 hour. After reaction, 800 µl of a mixture containing 150 mM NaCl, 1 mM EDTA and 20 mM tris-HCl (pH 8.0), 10 µl of 20 mg/ml proteinase K (Takara Shuzo) and 50 µl of 10% aqueous solution of sodium lauryl sulfate were added to the reaction mixture. The mixture was incubated at 37°C for 1 hour. After reaction, the mixture was subjected to phenol-chloroform extraction, ethanol precipitation and air-drying, and then dissolved in 50 µl of TE to obtain a genomic DNA solution.

(2) Cloning of RNase HII gene

The entire genomic sequence of the Archaeoglobus fulgidus has been published [Klenk, HP et al., Nature, 390:364-370 (1997)]. The existence of one gene encoding a homologue of RNase HII (AF0621) was known (SEQ ID NO:6, http: //www.tigr.org/tdb/CMR/btm/htmls/Splashpage.htlm).

Primers AfuNde (SEQ ID NO:7 and AfuBam (SEQ ID NO:8) were synthesized on the basis of the sequence of the AF0621 gene (SEQ ID NO:6).

A PCR was carried out using 30 ng of the Archaeoglobus fulgidus genomic DNA prepared in (1) as a template, and 20 pmol each of AfuNde and AfuBam as primers in a volume of 100 µl. Pyrobest DNA polymerase (Takara Shuzo) was used as a DNA polymerase for the PCR according to the attached protocol. The PCR was carried out as follows: 40 cycles of 94°C for 30 seconds, 55°C for 30 seconds and 72°C for 1 minute. An amplified DNA fragment of about 0.6 kb was digested with NdeI and BamHI (both from Takara Shuzo). Then, a plasmid pAFU204 was constructed by incorporating the resulting DNA fragment between NdeI and BamHI sites in a plasmid vector pTV119Nd (a plasmid in which the NcoI site in pTV119N is converted into a NdeI site).

(3) Determination of nucleotide sequence of DNA fragment containing RNase HII gene

The nucleotide sequence of the DNA fragment inserted into pAFU204 obtained in (2) was determined according to a dideoxy method.

Analysis of the determined nucleotide sequence revealed an open reading frame presumably encoding RNase HII. The nucleotide sequence of the open reading frame is shown in SEQ ID NO:9. The amino acid sequence of RNase HII deduced from the nucleotide sequence is shown in SEQ ID NO:10.

Escherichia coli JM109 transformed with the plasmid pAFU204 is designated and indicated as Escherichia coli JM109/pAFU204, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM P-18221 and transmitted to International Patent Organism Depositary, National Institute of Advanced Industrial Science and Technology under Budapest Treaty on August 2, 2001 (date of transmission to international depositary authority) under accession number FERM BP-7691.

(4) Preparation of purified RNase HII preparation

Escherichia coli JM109 was transformed with pAFU204 obtained in (2). The resulting Escherichia coli JM109 harboring pAFU204 was inoculated into 2 L of LB medium containing 100 µg/ml of ampicillin and cultured with shaking at 37°C for 16 hours. After cultivation, cells collected by centrifugation were suspended in 37.1 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 70 °C for 15 minutes. It was then centrifuged at 12000 rpm for 10 minutes again to collect a supernatant. Thus, 40.3 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

The flow-through RNase HII fraction was subjected to RESOURSE S column (Amersham Pharmacia Biotech) equilibrated with Buffer A and chromatographed using FPLC system (Amersham Pharmacia Biotech). As a result, RNase HII flowed through the RESOURSE S column.

40.0 ml of the flow-through RNase HII fraction was dialyzed against 2 L of Buffer B (50 mM tris-HCl (pH 7.0), 1 mM EDTA) containing 50 mM NaCl for 2 hours. The dialysis was repeated two more times. 40.2 ml of the dialyzed enzyme solution was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 240 mM NaCl was obtained.

7.8 ml of the RNase HII fraction was concentrated by ultrafiltration using Centricon-10 (Amicon). Four portions each separated from about 600 μl of the concentrate were subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HII was eluted at a position corresponding to a molecular weight of 30.0 kilodalton. This molecular weight corresponds to that of the RNase HII in the monomeric form. The RNase HII eluted as described above was used as Afu RNase HII preparation.

The enzymatic activity of the thus obtained Afu RNase HII preparation was measured as described in Referential Example 1-(4). As a result, an RNase H activity was observed for the Afu RNase HII preparation.

Unit value of a heat-resistant RNase H used in the method of the present invention was calculated as follows.

1 mg of poly(rA) or poly(dT) (both from Amersham Pharmacia Biotech) was dissolved in 1 ml of 40 mM tris-HCl (pH 7.7) containing 1 mM EDTA to prepare a poly(rA) solution and a poly(dT) solution.

The poly(rA) solution (to a final concentration of 20 μg/ml) and the poly(dT) solution (to a final concentration of 30 μg/ml) were then added to 40 mM tris-HCl (pH 7.7) containing 4 mM $MgCl_2$, 1 mM DTT, 0.003% BSA and 4% glycerol. The mixture was reacted at 37°C for 10 minutes and then cooled to 4°C to prepare a poly(rA)-poly(dT) solution. 1 μl of an appropriately diluted enzyme solution was added to 100 μl of the poly(rA)-poly(dT) solution. The mixture was reacted at 40°C for 10 minutes. 10 μl of 0.5 M EDTA was added thereto to terminate the reaction. Absorbance at 260 nm was then measured. As a control, 10 μl of 0.5 M EDTA was added to the reaction mixture, the resulting mixture was reacted at 40°C for 10 minutes, and the absorbance was then measured. A value (difference in absorbance) was obtained by subtracting the absorbance for the control from the absorbance for the reaction in the absence of EDTA. Thus, the concentration of nucleotide released from poly(rA)-poly(dT) hybrid by the enzymatic reaction was determined on the basis of the difference in absorbance. One unit of an RNase H was defined as an amount of enzyme that increases $A_{260}$ corresponding to release of 1 nmol of ribonucleotide in 10 minutes calculated according to the following equation:

Unit = [Difference in Absorbance x Reaction

Volume (ml)] / 0.0152 x (110/100) x Dilution Rate

Referential Example 3

Unit value of a mesophilic RNase H used in the method of the present invention was measured according to the following method.

(1) Preparation of reagent solutions used

Reaction mixture for determining activity: The following substances at the indicated final concentrations were contained in sterile water: 40 mM tris-hydrochloride (pH 7.7 at 37°C), 4 mM magnesium chloride, 1 mM DTT, 0.003% BSA, 4% glycerol and 24 μM poly(dT).

Poly[8-$^3$H]adenylic acid solution: 370 kBq of a poly[8-$^3$H]adenylic acid solution was dissolved in 200 μl of sterile water.

Polyadenylic acid solution: Polyadenylic acid was diluted to a concentration of 3 mM with sterile ultrapure water.

Enzyme dilution solution: The following substances at the indicated final concentrations were contained in sterile water: 25 mM tris-hydrochloride (pH 7.5 at 37°C), 5 mM 2-mercaptoethanol, 0.5 mM EDTA (pH 7.5 at 37°C), 30 mM sodium chloride and 50% glycerol.

Preparation of heat-denatured calf thymus DNA: 200 mg of calf thymus DNA was suspended and allowed to swell in 100 ml of TE buffer. The solution was diluted to a concentration of 1 mg/ml with sterile ultrapure water based on the absorbance measured at UV 260 nm. The diluted solution was heated at 100°C for 10 minutes and then rapidly cooled in an ice bath.

(2) Method for measuring activity

7 µl of the poly[8-$^3$H] adenylic acid solution was added to 985 µl of the reaction mixture for determining activity prepared in (1) above. The mixture was incubated at 37°C for 10 minutes. 8 µl of polyadenylic acid was added to the mixture to make the final concentration to 24 µM. The mixture was further incubated at 37°C for 5 minutes. Thus, 1000 µl of a poly[8-$^3$H]rA-poly-dT reaction mixture was prepared. 200 µl of the reaction mixture was then incubated at 30°C for 5 minutes. 1 µl of an appropriate serial dilution of an enzyme solution was added thereto. 50 µl each of samples was taken from the reaction mixture over time for use in subsequent measurement. The period of time in minutes from the addition of the enzyme to the sampling is defined as Y. 50 µl of a reaction mixture for total CPM or for blank was prepared by adding 1 µl of the enzyme dilution solution instead of an enzyme solution. 100 µl of 100 mM sodium pyrophosphate, 50 µl of the heat-denatured calf thymus DNA solution and 300 µl of 10% trichloroacetic acid (300 µl of ultrapure water for measuring total CPM) were added to the sample. The mixture was incubated at 0°C for 5 minutes, and then centrifuged at 10000 rpm for 10 minutes. After centrifugation, 250 µl of the resulting supernatant was placed in a vial. 10 ml of Aquasol-2 (NEN Life Science Products) was added thereto. CPM was measured in a liquid scintillation counter.

(3) Calculation of units

Unit value for each enzyme was calculated according to the following equation:

$$\text{Unit/ml} = \{(\text{measured CPM - blank CPM}) \times 1.2^{*} \times 20 \times 1000 \times \text{dilution rate}\}\ 200\ (\mu l) / (\text{total CPM} \times Y\ (\text{min.}) \times 50\ (\mu l) \times 9^{**})$$

1.2*: Amount in nmol of poly[8-$^3$H]rA-poly-dT contained in total CPM per 50 µl.
9**: Correction coefficient.

Example 1

(1) Extraction of DNA from sputum

A sputum was processed according to the NALC method recommended by CDC (Centers for Diseases Control and Prevention), U.S.A. Specifically, a sputum was placed in a 50-ml screw-capped tube. An equal volume of an NALC solution (prepared by adding 0.5 g of N-acetyl-L-cysteine to a mixture of 50 ml of 0.1 M sodium citrate and 50 ml of 4% sodium hydroxide) was added thereto. The mixture was stirred adequately for 20 seconds using a tube mixer to make the test sample fluidal. After allowing to stand at room temperature for 15 minutes, phosphate buffered saline (PBS) was added to a total volume of 50 ml. Then, a precipitate was collected by centrifugation at 3000 x g or more for 20 minutes. 50 µl of a lysis buffer (10 mM HEPES buffer (pH 7.8) containing 1 unit of mutanolysin (Sigma)) was added to the precipitate and mixed adequately. The mixture was reacted at 37°C for 30 minutes, and ten heated for five minutes in boiling water or a heat block at 96°C. Optionally, a supernatant was obtained by centrifugation using a microcentrifuge. The supernatant prepared as described above was used as a DNA extract from the sputum in subsequent procedures. In addition, Dr. GenTLE (from yeast) (Takara Shuzo) was also used for nucleic acid extraction according to the attached instructions.

(2) Preparation of probe and chimeric oligonucleotide primer

A specific oligonucleotide probe for detecting IS 6110 gene from a Mycobacterium tuberculosis complex was prepared on the basis of the nucleotide sequence of GenBank Accession No. X52471. Specifically, an oligonucleotide probe MTIS-2BF (SEQ ID NO:11) having fluorescein isothiocyanate (FITC) at the 5' end was synthesized using a DNA synthesizer.
Chimeric oligonucleotide primers for synthesizing a DNA fragment derived from Mycobacterium tuberculosis complex IS 6110 gene using the ICAN method were prepared. Specifically, a forward primer MTIS-2F (SEQ ID NO: 13) and a reverse primer MTIS2-RBio (SEQ ID NO:14) having biotin at the 5' end were synthesized on the basis of the nucleotide sequence of IS 6110 gene from a Mycobacterium tuberculosis complex (SEQ ID NO:12) using a DNA synthesizer. Similarly, a forward primer K-F1033-2 (SEQ ID NO:15) and a reverse primer K-R1133-2Bio (SEQ ID NO:

16) having biotin at the 5' end were synthesized using a DNA synthesizer.

(3) Amplification by ICAN method

A reaction mixture for ICAN reaction as shown in Table 1 was prepared and incubated at 60°C for 30 minutes.

Table 1

| Component | Volume (µl) |
|---|---|
| 5 x reaction buffer (pH 7.8) | 10 |
| 100 mM magnesium acetate | 2 |
| 10 mM dNTPs (Takara Shuzo) | 2.5 |
| Primer MTIS-2F (100 µM) | 0.5 |
| Primer MTIS-2RBio (100 µM) | 0.5 |
| Pfu RNase HII (63 ng/µl) | 0.5 |
| BcaBEST DNA polymerase (Takara Shuzo, 8U/µl) | 0.5 |
| DNA extract | 1 |
| $H_2O$ | 32.5 |
| Total | 50 µl |

Composition of 5 x reaction buffer:

100 mM HEPES-potassium hydroxide (pH 7.8)
500 mM potassium acetate
5% dimethyl sulfoxide (DMSO)
0.05% bovine serum albumin (BSA)

Similarly, an ICAN amplification reaction was also carried out using a combination of the primers K-F1033-2/K-R1133-2. After reaction, a portion of the reaction mixture was subjected to electrophoresis to confirm the amplified fragment of interest.

(4) Detection by solid-phase plate luminescence method

Streptavidin (Nacalai Tesque) was dissolved in PBS at a concentration of 2 µg/ml, and 150 µl of the resulting solution was added to each well of a 96-well microtiter plate for white luminescence detection. The plate was allowed to stand at 4°C overnight for immobilization. After discarding the streptavidin solution, 200 µl of a 1% BSA-PBS solution was dispensed to each well, and the plate was allowed to stand at 4°C overnight for blocking. The solution was discarded, and the thus obtained plate was used in subsequent experiments as a streptavidin-coated plate.

50 µl of a hybridization buffer (5 x SSC containing 1% Triton X-100 and 1% BSA) and 10 µl of an ICAN reaction mixture containing a amplified fragment obtained using a combination of primers MTIS-2F/MTIS2-RBio in Example 1-(3) was added to each well of the streptavidin-coated plate. The mixture was mixed adequately and reacted at room temperature for 15 minutes for entrapping onto the surface of the plate. Next, 5 µl of a DNA denaturation solution (0.1 N NaOH) was added to each well. The resulting mixture was mixed adequately and subjected to DNA denaturation for three minutes to denature the double-stranded DNAs entrapped on the surface of the plate into single-stranded DNAs. The above-mentioned probe MTIS2BF was diluted with the hybridization buffer to a concentration 5 pmol/ml, and 100 µl of the dilution was added to each well. The mixture was mixed adequately and reacted at room temperature for 40 minutes. The pH of each of the wells was 13-14. After reaction, the solution in the well was discarded. The well was washed twice with 200 µl/well of a washing buffer (25 mM Tris-hydrochloride (pH 7.5), 150 mM NaCl, 1% BSA, 0.05% Tween 20). Subsequently, 100 µl of a solution containing a peroxidase-labeled anti-FITC rabbit antibody (Chemicon) at a concentration of 2 µg/ml in the hybridization buffer was added to each well. The mixture was reacted at room temperature for 20 minutes. After reaction, the mixture was discarded, the well was washed four times with 200 µl/well of the washing buffer, and 100 µl of a luminescent substrate SuperSignal ELISA Pico Substrate (Pierce) was added to each well. Immediately, the plate was subjected to measurement of relative luminescence intensity using a luminescence plate reader (Labsystems).

(5) Examination of detection sensitivity

Samples containing a DNA derived from a sputum corresponding to 0, 5 or 10 copies of a Mycobacterium tuberculosis complex genome were subjected to detection of a Mycobacterium tuberculosis complex DNA as described above. As a result, strong luminescence with an S/N ratio of about 300 could be detected for the sample containing 5 copies of the genome as shown in Table 2.

Table 2

| Genome | Luminescence intensity (RLU) |
|---|---|
| 0 copy | 11 |
| 5 copies | 3078 |
| 10 copies | 6287 |

As described above, it was demonstrated that the present invention enables a rapid and highly sensitive test for a Mycobacterium tuberculosis complex. Furthermore, it was confirmed that both the nucleic acid extraction method of the present invention and the method in which the reagent for Dr. GenTLE from yeast was used could be suitably utilized for the method for detecting a Mycobacterium tuberculosis complex of the present invention. Thus, it was demonstrated that the nucleic acid extraction method of the present invention was useful in view of the convenient operativity.

Example 2

Sputa collected from 26 patients suspected to be infected with a Mycobacterium tuberculosis complex were subjected to detection of a Mycobacterium tuberculosis complex DNA as described in Example 1. In addition, the same test samples were subjected to detection using Amplicor Mycobacterium tuberculosis (Roche) which is a conventional PCR-based kit in which a DNA encoding 16S rRNA is used as a target, and a test for Mycobacterium tuberculosis complex according to a cultivation method using the Ogawa medium. The results are shown in Table 3.

Table 3

| Ogawa cultivation method | Positive case (n=9) | | | | Negative case (n=17) | | | |
|---|---|---|---|---|---|---|---|---|
| Gene Amplification method | PCR | | Method of the invention | | PCR | | Method of the invention | |
| | P* | N* | P* | N* | P* | N* | P* | N* |
| | 8 | 1 | 9 | 0 | 2 | 15 | 0 | 17 |

*P: positive; N: negative.

As shown in Table 3, the results obtained using the method of the present invention were consistent well with those obtained using the cultivation test. According to the PCR method, two cases that were determined to be negative according to the cultivation method were determined to be false positive, while one case that was determined to be positive according to the cultivation method was determined to be false negative.

Thus, it was confirmed that accurate test results could be obtained very rapidly and conveniently using the method of the present invention as compared with the conventional methods.

Example 3

The specificity of the detection method of the present invention for Mycobacterium tuberculosis complex was examined. Genomic DNAs from 37 strains listed in Table 4 were used.

Table 4

| Mycobacterium | | Streptococcus | |
|---|---|---|---|
| GTC No.603 | M.avium | 1234 | S.agalactiae |
| 857 | M.abscessus | 1700 | S.mutans |
| 499 | M.bovis BCG | 261 | S.pneumoniae |
| 858 | M.chelonae | 262 | S.pyogenes |
| 609 | M.fortuitum | 217 | S.sanguinis |
| 610 | M.gastri | Staphylococcus | |
| 612 | M.gordonae | GTC No.286 | S.aureus subsp. aureus |
| 613 | M.intracellulare | 289 | S.epidermidis |
| 614 | M.kansasii | 266 | S.intermedius |
| 616 | M.marinum | 265 | S.saprophyticus |
| 617 | M.nonchromogenicum | Enterococcus | |
| 1725 | M.peregrinum | 228 | E.faecalis |
| 619 | M.scrofulaceum | 227 | E.faecium |
| 620 | M.simiae | Peptostreptococcus | |
| 622 | M.szulgai | 200 | P.magnus |
| 623 | M.terrae | Stomatococcus | |
| 624 | M.triviale | 311 | S.mucilaginosus |
| 601 | M.tuberculosis | Corynebacterium | |
| 626 | M.xenopi | 1438 | C.pseudotuberculosis |
| Pseudomonas | | Listeria | |
| 2 | P.aeruginosa | 149 | L.monocytogenes |
| Escherichia | | Erysipelothrix | |
| 503 | E.coli | 555 | E.rhuthiopathiae |

The copy numbers of the respective genomic DNAs were calculated based on the OD values, and dilutions each containing 2 x $10^7$ copies were prepared. The genomic DNA was used as a template for detection using a reaction mixture having the composition as shown in Table 5.

Table 5

| Component | Volume (µl) |
|---|---|
| 5 x reaction buffer (pH 7.8) | 10 |
| 100 mM magnesium acetate | 2 |
| 10 mM dNTPs (Takara Shuzo) | 2.5 |
| Primer MTIS-2F (100 µM) | 0.5 |
| Primer MTIS-2RBio (100 µM) | 0.5 |
| Afu RNase HII (17.5 U/µl) | 0.5 |
| BcaBEST DNA polymerase (16 U/µl) | 0.5 |
| DNA extract | 1 |
| $H_2O$ | 32.5 |
| Total | 50 µl |

The reaction mixture was incubated at 60°C for one hour. Detection was carried out as described in Example 1(4). As a result, only the Mycobacterium tuberculosis strain and the Mycobacterium bovis BCG strain could be specifically detected. Based on these results, it was confirmed that the method of the present invention was highly specific.

Example 4

(1) Detection of a Mycobacterium tuberculosis complex using magnetic beads was examined. The primers MTIS-2F and MTIS-2RBio were used as primers. As templates, 30-, 300- and 3000-fold dilutions of the genome extracted from the positive test sample in Example 2 with sterile water were prepared. The reaction was carried out as follows. Briefly, at final concentrations, 32 mM HEPES-potassium hydroxide buffer (pH 7.8), 100 mM potassium acetate, 1% DMSO, 0.01% BSA, 4 mM magnesium acetate, 500 µM each of dNTPs, 50 pmol each of the primers MTIS-2F and MTIS-2R, 8.75 U of Afu RNase H, 8 U of BcaBEST DNA polymerase and 1 µl of the template as well as sterile water to a final volume of 50 µl were mixed. The reaction mixture was placed in Thermal Cycler Personal which had been set at 60°C and incubated for 60 minutes. The resulting amplified fragment was subjected to detection using streptavidin-coated magnetic beads (Pierce) in an automated detection instrument Lumipuls (Fujirebio). Magnetic beads having streptavidin capable of binding 100 pmol of biotin being immobilized were reacted with the biotinylated amplified fragment for 5 minutes on the first layer of a cuvette. Then, 0.1 N NaOH was added thereto. Hybridization with the FITC-labeled probe MTISBF was carried out for 5 minutes. After washing, a POD-labeled anti-FITC antibody was added thereto. After reacting for 5 minutes and washing, a luminescent substrate was added thereto. As a result, it was shown that detection can be carried out semi-quantitatively in a short time (20 minutes) using magnetic beads in a conventional automated detection instrument. The reagents as described in Example 1 were used. The detection was carried out by measuring the luminescence level by photocounting. The results are shown in Table 6.

Table 6

| Template | Photocounting | S/N ratio |
|---|---|---|
| x 30 | $3.55 \times 10^7$ | 29.6 |
| x 300 | $1.21 \times 10^7$ | 10.0 |
| x 3000 | $0.21 \times 10^7$ | 1.75 |
| 0 | $0.12 \times 10^7$ | - |

Based on the results as shown in Table 6, it was confirmed that detection could be carried out with a sensitivity equivalent to that of the conventional plate luminescence method.

(2) A detection system using an internal control (IC) in combination was examined. The internal control was prepared as follows. Briefly, PCR amplification was carried out using a human genomic DNA as a template and primers of SEQ ID NOS:17 and 18. The resulting amplified fragment was purified according to a conventional method, and inserted into pT7 Blue T-vector (Novagen). The plasmid was used as an internal control. The reaction was carried out as described in Example 3, and 1, 3 or 10 pg of the plasmid was added. As templates, 0, 2 or 20 copies of a Mycobacterium tuberculosis complex genome were used. A probe for IC (SEQ ID NO:19) having a FITC label at the 5' end (ICF probe) and the FITC-labeled probe MTISBF were used for detection. As a result, the Mycobacterium tuberculosis complex genome could detected using the IC plasmid at each concentration. In particular, it was confirmed that use of 3 pg of the IC plasmid was preferable.

(3) A hybrid chromatography method as a method for detecting the above-mentioned amplified fragment was examined. Streptavidin (Nacalai Tesque) was immobilized onto a nitrocellulose membrane. A water-absorptive pad was connected therewith to construct a hybrid chromatography strip. This strip was used to detect the amplified fragment obtained in Example 1 according to a hybrid chromatography method. Detection was carried out by color development using 1-step TMB-Blotting (Pierce). Specifically, a reaction mixture containing the amplified fragment was developed on the nitrocellulose membrane. Then, a 0.1 N NaOH solution, an FITC-labeled probe, a washing solution and a color development solution were developed in this order. As a result, a blue band was detected for the Mycobacterium tuberculosis complex-positive amplified fragment. It was confirmed that this method is useful as a rapid genetic test method because results are obtained with naked eyes in 5 to 10 minutes after conducting the method of the present invention.

Example 5

A kit for Mycobacterium tuberculosis complex was constructed on the basis of the results obtained in Examples 1 to 4. The respective components are shown below:

| (1) Reagent for ICAN amplification (50 reactions) | |
|---|---|
| 5 x reaction buffer | 500 µl |
| 100 mM magnesium acetate | 100 µl |
| 10 mM dNTP mix | 125 µl |
| 0.1 mM MTIS-2F primer | 25 µl |
| 0.1 mM MTIS-SRBio primer | 25 µl |
| Afu RNase HII (17.5 U/µl) | 25 µl |

(continued)

| (1) Reagent for ICAN amplification (50 reactions) | |
|---|---|
| BcaBEST DNA polymerase (16 U/μl) | 25 μl |

| (2) Reagent for detection (50 reactions) | |
|---|---|
| Streptavidin-immobilized 96-well plate | 50 plates |
| Hybridization buffer (5 x SSC, 1% Triton X100, 1% BSA) | 2.5 ml |
| Denaturation agent (0.1 N NaOH) | 0.5 ml |
| Detection MT probe solution (25 nM MTIS-2BF probe) | 5 ml |
| Detection IC probe solution (25 nM ICF probe) | 5 ml |
| Labeled antibody solution (BlockAce (Dainippon Pharmaceutical) : PBS =1 : 3, 500 units of POD-anti-FITC antibody) | 5 ml |
| 10 x washing solution (250 mM tris buffer (pH7.5), 1.5 M NaCl, 1% BSA, 0.5% Tween 20) | 20 ml |
| Luminescence reagent A . (SuperSignal ELISA Pico Substrate A solution (Pierce)) | 2.5 ml |
| Luminescence reagent B (SuperSignal ELISA Pico Substrate B solution (Pierce)) | 2.5 ml |
| Internal control solution (IC plasmid, TE buffer) | 0.1 ml |
| Positive control solution (IS 6110-containing plasmid, TE buffer) | 0.02 ml |
| Negative control solution (TE buffer) | 0.02 ml |

The above-mentioned reagents were used to detect the Mycobacterium tuberculosis strain and the Mycobacterium bovis BCG strain. As a result, it was confirmed that they could be detected rapidly, sensitively and specifically.

Example 6

(1) Extraction of DNA from swab test sample

An extraction buffer containing a detergent (a test sample treatment reagent for Amplicor STD (Roche)) was added to swab test samples taken by rubbing with cotton swabs from a male urethra and a female uterine cervix. The mixtures were heated at 95°C to 100°C for 10 minutes.

(2) Preparation of probe and chimeric oligonucleotide primer

A specific oligonucleotide probe for detecting cryptic plasmid from a chlamydia was prepared. Specifically, an oligonucleotide probe CT1234 (SEQ ID NO:20) having fluorescein isothiocyanate (FITC) at the 5' end was synthesized using a DNA synthesizer. In addition, a specific oligonucleotide probe for detecting cppB gene from a gonococcus was prepared. Specifically, an oligonucleotide probe CppB3 (SEQ ID NO:21 having fluorescein isothiocyanate (FITC) at the 5' end was synthesized using a DNA synthesizer.

Chimeric oligonucleotide primers for synthesizing a DNA fragment derived from cryptic plasmid from a chlamydia using the ICAN method were prepared. Specifically, forward primers F1212-22 (SEQ ID NO:23) and F1162-22 (SEQ ID NO:24) as well as reverse primers R1272-22Bio (SEQ ID NO:25) and R1379-22Bio (SEQ ID NO:26) each having biotin at the 5' end were synthesized on the basis of the nucleotide sequence of cryptic plasmid from a chlamydia (SEQ ID NO:22) using a DNA synthesizer.

Chimeric oligonucleotide primers for synthesizing a DNA fragment derived from cppB gene from a gonococcus using the ICAN method were prepared. Specifically, a forward primer pJDBF-1 (SEQ ID NO:28) and a reverse primer pJDBR-3Bio (SEQ ID NO:29) having biotin at the 5' end were synthesized on the basis of the nucleotide sequence of cryptic plasmid from a chlamydia (SEQ ID NO:27) using a DNA synthesizer.

(3) Amplification by ICAN method

A reaction mixture for ICAN method as shown in Table 7 was prepared and incubated at 55°C for 60 minutes.

Table 7

| Component | Volume (µl) |
|---|---|
| 5 x reaction buffer (pH 7.8) | 10 |
| 100 mM magnesium acetate | 2 |
| 10 mM dNTPs (Takara Shuzo) | 2.5 |
| Primer F1212-22 (100 µM) | 0.5 |
| Primer R1272-22Bio (100 µM) | 0.5 |
| Primer pJDBF-1 (100 µM) | 0.5 |
| Primer pJDBR-3 (100 µM) | 0.5 |
| Pfu RNase HII (63 ng/µl) | 0.5 |
| BcaBEST DNA polymerase (Takara Shuzo, 8 U/µl) | 0.5 |
| DNA extract | 1 |
| $H_2O$ | 32.5 |
| Total | 50 µl |

Composition of 5 x reaction buffer:

100 mM HEPES-potassium hydroxide (pH 7.8)
500 mM potassium acetate
5% dimethyl sulfoxide (DMSO)
0.05% bovine serum albumin (BSA)

After reaction, a portion of the reaction mixture was subjected to electrophoresis, and the amplified fragment of interest was observed.

(4) Detection by solid-phase plate luminescence method

Streptavidin (Nacalai Tesque) was dissolved in PBS at a concentration of 2 µg/ml, and 150 µl of the resulting solution was added to each well of a 96-well microtiter plate for white luminescence detection. The plate was allowed to stand at 4°C overnight for immobilization. After discarding the streptavidin solution, 200 µl of a 1% BSA-PBS solution was dispensed to each well, and the plate was allowed to stand at 4°C overnight for blocking. The solution was discarded, and the thus obtained plate was used in subsequent experiments as a streptavidin-coated plate.

50 µl of a hybridization buffer (5 x SSC containing 1% Triton X-100 and 1% BSA) was added to each well of the streptavidin-coated plate. 10 µl of an ICAN reaction mixture containing a amplified fragment obtained using combinations of primers F1212-22/R1272-22Bio and pJDBF-1/pJDBR-3Bio in Example 1-(3) was added to each well (two wells for one test sample). The mixture was mixed adequately and reacted at room temperature for 15 minutes for entrapping onto the surface of the plate. Next, 5 µl of a DNA denaturation solution (0.1 N NaOH) was added to each well. The resulting mixture was mixed adequately and subjected to DNA denaturation for three minutes to denature the double-stranded DNAs entrapped on the surface of the plate into single-stranded DNAs. The above-mentioned probe CT1234 or CppB3 was diluted with the hybridization buffer to a concentration 5 pmol/ml, and 100 µl of the dilution was added to each well. The mixture was mixed adequately and reacted at room temperature for 40 minutes. The pH of each of the wells was 13-14. After reaction, the solution in the well was discarded. The well was washed twice with 200 µl/well of a washing buffer (25 mM Tris-hydrochloride (pH 7.5), 150, mM NaCl, 1% BSA, 0.05% Tween 20). Subsequently, 100 µl of a solution containing a peroxidase-labeled anti-FITC rabbit antibody (Chemicon) at a concentration of 2 µg/ml in the hybridization buffer was added to each well. The mixture was reacted at room temperature for 20 minutes. After reaction, the mixture was discarded, the well was washed four times with 200 µl/well of the washing buffer, and 100 µl of a luminescent substrate SuperSignal ELISA Pico Substrate (Pierce) was added to each well. Immediately, the plate was subjected to measurement of relative luminescence intensity using a luminescence plate reader (Labsystems).

(5) Examination of detection sensitivity

Samples containing a DNA corresponding to 0, 10 or 100 copies of a chlamydia genome were subjected to detection of a chlamydia DNA as described above. As a result, strong luminescence with an S/N ratio of about 30 could be detected for the sample containing 10 copies of the genome as shown in Table 8. Samples containing a DNA corresponding to 0, 10 or 100 copies of a gonococcus genome were subjected to detection of a gonococcus DNA as described above. As a result, strong luminescence with an S/N ratio of about 300 could be detected for the sample containing 10 copies of the genome as shown in Table 9.

Table 8

| Genome | Luminescence intensity (RLU) |
|---|---|
| 0 copy | 11 |
| 10 copies | 347 |
| 100 copies | 3961 |

Table 9

| Genome | Luminescence intensity (RLU) |
|---|---|
| 0 copy | 11 |
| 10 copies | 3026 |
| 100 copies | 3840 |

As described above, it was demonstrated that the present invention enables rapid and highly sensitive tests for a chlamydia and a gonococcus.

Example 7

Simultaneous amplification and detection of a chlamydia DNA and a gonococcus DNA from 12 test samples suspected to have mixed infections with a chlamydia and a gonococcus were carried out according to the procedure as described in Example 1.

The results were as follows: positive only for a chlamydia (5 cases); positive only for a gonococcus (1 case); positive for both a chlamydia and a gonococcus (3 cases); and negative for both (3 cases). These results were consistent with those obtained using a conventional PCR kit (Amplicor STD (Roche)).

Example 8

Preparation of primer and probe

Chimeric oligonucleotide primers HCV-F (SEQ ID NO:30) and HCV-R1 (SEQ ID NO:31) as well as oligonucleotide primers for reverse transcription reaction (SEQ ID NOS:32 and 33) were synthesized on the basis of the nucleotide sequence of HCV genome. In addition, oligonucleotide probes (SEQ ID NOS:34 and 35) were synthesized. Oligonucleotide probes having TAMRA (N,N,N',N'-tetramethyl-6-carboxy-rhodamine, ABI) at the 5' ends were prepared using an automated DNA synthesizer and used as fluorescence-labeled probes.

(2) Preparation of synthetic RNA

A copy number was determined for an HCV-RNA-positive serum obtained after obtaining informed consent using Amplicor HCV Monitor kit (Roche). Serial 10-fold dilutions with a test sample dilution solution attached to the kit at concentrations ranging from 1 copy/µl to 1 x $10^7$ copies/µl were prepared and used as HCV-RNAs.

(3) Reverse transcription reaction

A cDNA was synthesized from the HCV-RNA prepared in (2) above using the primer for reverse transcription reaction prepared in (1) above and First-strand cDNA Synthesis Kit (Takara Shuzo).

(4) Preparation of ICAN reaction mixture.

An ICAN reaction was carried out using the cDNA solution. The composition of the reaction mixture in a tube is shown in Table 10.

Table 10

| Component | Volume |
|---|---|
| cDNA solution | 3 μl |
| Injectable distilled water | 28.75 μl |
| 5 x Hepes Buffer | 10 μl |
| 10 mM dNTP Mixture | 2.5 μl |
| 100 μM Forward primer | 0.5 μl |
| 100 μM Reverse primer | 0.5 μl |
| Bca BEST DNA polymerase (22 U/μl) | 0.25 μl |
| PFU RNase H (60 U/μl) | 0.5 μl |
| 0.5% propylenediamine | 2 μl |
| 100 mM magnesium acetate | 2 μl |

(5) Homogeneous detection

47 μl of the reaction mixture was added to each tube. 3 μl of the cDNA solution prepared in (4) was added thereto. The tube was incubated at 56°C for 30 minutes. After reaction, the fluorescence-labeled probe prepared in (1) above was added to the reaction mixture at a final concentration of 300 nM. After treatment at 98°C for two minutes, the mixture was cooled to 25°C by ice cooling and incubated at the temperature. The reaction mixture was subjected to measurement of fluorescence intensity using a fluorescence detector Fluoroscan (Labsystems). In addition, a commercially available PCR-based Amplicor HCV kit (Roche) was used for measurement of the same test sample as a control. The results are shown in Figure 1. Figure 1 is a graph illustrating the change in fluorescence intensity as well as the comparison of the measurement range with the conventional method when measurements were carried out at varying HCV-RNA concentrations. The longitudinal axis on the left represents the OD450 value; the longitudinal axis on the right represents the fluorescence intensity (SN ratio); and the horizontal axis represents the amount of HCV-RNA. In Figure 1, closed boxes represent results obtained using the method of the present invention and closed circles represent results obtained using Amplicor HCV kit.

As shown in Figure 1, it was confirmed that more fluorescence intensity (SN ratio) was observed as the copy number of HCV-RNA was increased according to the method of the present invention. The total time required for the HCV detection method of the present invention was 45 minutes. On the other hand, Amplicor HCV kit as a control required about five hours. Regarding the HCV-RNA detection range, it was confirmed that the absorbance was increased as the copy number of HCV-RNA was increased using Amplicor HCV kit as shown in Figure 1. However, the measurement range was two orders of magnitude and the measurement was not quantitative for samples containing $10^5$ copies or more. On the other hand, the method of the present invention was confirmed to result in a broad detection range of three orders of magnitude. Furthermore, it was confirmed that the method of the present invention is superior in the convenience and rapidity and, therefore, the method is suitable for handling a large number of test samples.

Example 9

(1) Preparation of HCV-RNA from patient's serum

Detection of HCV from a clinical test sample was examined. An RNA was prepared from 100 μl each of serum test samples from 28 patients with HCV obtained after obtaining informed consent using an HCV-RNA extraction reagent attached to Amplicor HCV kit (Roche).

(2) Detection of HCV-RNA in patient's serum

HCV-RNA was amplified according to the procedure as described in Example 1(3) and (4). and subjected to the detection method of the present invention. In this Example, a cutoff value was defined as a mean fluorescence intensity value obtained from ten negative control experiments using a sample without HCV-RNA as control ± 3 SD. (three times standard deviation). A test sample resulting in fluorescence intensity over the cutoff value was determined to be positive. On the other hand, the same test sample was subjected to measurement using the commercially available Amplicor HCV kit to determine whether the sample was positive or negative according to the instructions attached to the kit. Results of comparison between the detection method of the present invention and Amplicor HCV kit as a conventional method are shown in Table 11.

Table 11

| | Method of the present invention | |
|---|---|---|
| | Positive | Negative |
| Amplicor method | | |
| Positive | 18 cases | 0 case |
| Negative | 0 case | 10 cases |

As shown in Table 11, it was confirmed that the measurement results obtained using the method of the present invention were correlated well with those obtained using the conventional method. It was further confirmed that the measurement according to the method of the present invention is more sensitive, more rapid and more convenient than that according to the conventional method.

Industrial Applicability

The present invention provides a method, a primer and a probe as well as a kit that enable sensitive, specific, rapid and convenient measurement of a pathogenic microorganism, and that can be used to handle a number of test samples in a defined period of time without the use of special equipment.

Sequence Listing Free Text

SEQ ID NO:2: PCR primer 1650Nde for cloning a gene encoding a polypeptide having a RNaseHII activity from Pyrococcus furiosus
SEQ ID NO:3: PCR primer 1650Bam for cloning a gene encoding a polypeptide having a RNaseHII activity from Pyrococcus furiosus
SEQ ID NO:7: PCR primer AfuNde for cloning a gene encoding a polypeptide having a RNaseHII activity from Archaeoglobus fulgidus
SEQ ID NO:8: PCR primer AfuBam for cloning a gene encoding a polypeptide having a RNaseHII activity from Archaeoglobus fulgidus
SEQ ID NO:11: Oligonucleotide probe to detect the DNA derived from Mycobacterium tuberculosis
SEQ ID NO:13: Chymeric oligonucleotide primer to amplify the DNA fragment of IS 6110 gene derived from Mycobacterium tuberculosis."nucleotides 16 to 18 are ribonucleotides-other nucleotides are deoxyribonucleotides"
SEQ ID NO:14: Chymeric oligonucleotide primer to amplify the DNA fragment of IS 6110 gene derived from Mycobacterium tuberculosis."nucleotides 19 to 21 are ribonucleotides-other nucleotides are deoxyribonucleotides"
SEQ ID NO:15: Chimeric oligonucleotide primer to amplify the DNA fragment of IS 6110 gene derived from Mycobacterium tuberculosis." nucleotides 19 to 21 are ribonucleotides-other nucleotides are deoxyribonucleotides"
SEQ ID NO:16: Chimeric oligonucleotide primer to amplify the DNA fragment of IS 6110 gene derived from Mycobacterium tuberculosis."nucleotides 20 to 22 are ribonucleotides-other nucleotides are deoxyribonucleotides"
SEQ ID NO:17: Oligonucleotide primer C4-MT2F-S100 to amplify the DNA fragment of metaroprotease gene from human
SEQ ID NO:18: Oligonucleotide primer C4-MT2R-A to amplify the DNA fragment of metaroprotease gene from human
SEQ ID NO:19: Oligonucleotide probe to detect the internal control DNA
SEQ ID NO:20: Oligonucleotide probe CT1234 to detect the Chramydia criptic plasmid
SEQ ID NO:21: Oligonucleotide probe CppB3 to detect Neisseria gonorrhoeae cppB gene
SEQ ID NO:23: Chimeric oligonucleotide primer to amplify the DNA fragment of Chramydia criptic plasmid. "nucleotides 20 to 22 are ribonucleotides-other nucleotides are deoxyribonucleotides"
SEQ ID NO:24: Chimeric oligonucleotide primer to amplify the DNA fragment of Chramydia criptic plasmid. "nucleotides 20 to 22 are ribonucleotides-other nucleotides are deoxyribonucleotides"
SEQ. ID NO:25: Chimeric oligonucleotide primer to amplify the DNA fragment of Chramydia criptic plasmid. "nucleotides 20 to 22 are ribonucleotides-other nucleotides are deoxyribonucleotides"
SEQ ID NO:26: Chimeric oligonucleotide primer to amplify the DNA fragment of Chramydia criptic plasmid. "nucleotides 20 to 22 are ribonucleotides-other nucleotides are deoxyribonucleotides"
SEQ ID NO:28: Chimeric oligonucleotide primer to amplify the DNA fragment of Neisseria gonorrhoeae cppB gene. "nucleotides 18 to 20 are ribonucleotides-other nucleotides are deoxyribonucleotides"
SEQ ID NO:29: Chimeric oligonucleotide primer to amplify the DNA fragment of Neisseria gonorrhoeae cppB gene. "nucleotides 15 to 17 are ribonucleotides-other nucleotides are deoxyribonucleotides".
SEQ ID No:30: Designed chimeric oligonucleotide primer designated as HCV-F to amplify a portion of HCV. "nucleotides 19 to 21 are ribonucleotides-other nucleotides are deoxyribonucleotides"
SEQ ID No:31: Designed chimeric oligonucleotide primer designated as HCV-R3 to amplify a portion of HCV. "nucleotides 16 to 18 are ribonucleotides-other nucleotides. are deoxyribonucleotides"
SEQ ID No 32:Designed oligonucleotide primer to synthsize cDNA of HCV
SEQ ID No:33:Designed oligonucleotide primer to synthsize cDNA of HCV
SEQ ID No:34: Designed oligonucleotide probe to detect a DNA fragment amplifying a portion of HCV
SEQ ID No:35: Designed oligonucleotide probe to detect a DNA fragment amplifying a portion of HCV
SEQ ID No:36: Oligonucleotide primer to amplify the DNA fragment of IS 6110 gene derived from Mycobacterium tuberculosis
SEQ ID No:37: Oligonucleotide primer to amplify the DNA fragment of IS 6110 gene derived from Mycobacterium tuberculosis
SEQ ID No:41: Primer area to amplify a portion of HCV
SEQ ID No:42: Primer area to amplify a portion of HCV
SEQ ID No:43: Probe area to detect a DNA fragment amplifying a portion of HCV

## Claims

1. A probe containing the nucleotide sequence of SEQ ID NO:39 or a part thereof, which can be used to detect a Mycobacterium tuberculosis complex Mycobacterium tuberculosis, Mycobacterium bovis BCG, Mycobacterium africanum, Mycobacterium microti and/or Mycobacterium canetti.

2. A probe consisting of the nucleotide sequence of SEQ ID NO:11, which can be used to detect a Mycobacterium tuberculosis complex Mycobacterium tuberculosis, Mycobacterium bovis BCG, Mycobacterium africanum, Mycobacterium microti and/or Mycobacterium canetti.

3. A probe containing the nucleotide sequence of SEQ ID NO:27 or a part thereof, which can be used to detect a gonococcus Neisseria gonorrhoeae.

4. A probe consisting of the nucleotide sequence of SEQ ID NO:21, which can be used to detect a gonococcus Neisseria gonorrhoeae.

5. A probe containing the nucleotide sequence of SEQ ID NO:22 or a part thereof, which can be used to detect a chlamydia Chlamydia trachomatis.

6. A probe consisting of the nucleotide sequence of SEQ ID NO:20, which can be used to detect a chlamydia Chlamydia trachomatis.

7. A probe consisting of the nucleotide sequence of SEQ ID NO:34 or 35, which can be used to detect hepatitis C virus (HCV).

8. A probe that can hybridize to a target nucleic acid from a pathogenic microorganism at alkaline pH.

9. The probe according to claim 8, which can hybridize to a target nucleic acid from a pathogenic microorganism at alkaline pH of 8 to 14.

10. The probe according to claim 8 or 9, wherein the pathogenic microorganism is a Mycobacterium tuberculosis complex, a gonococcus, a chlamydia or HCV.

11. The probe according to claim 10, wherein the target nucleic acid from a pathogenic microorganism is selected from a nucleotide sequence of IS 6110 gene from a Mycobacterium tuberculosis complex, a nucleotide sequence of cppB gene from a gonococcus, a nucleotide sequence of pLGV440 from a chlamydia or a nucleotide sequence of the 5' untranslated region from HCV.

12. The probe according to claim 11, which contains the nucleotide sequence of SEQ ID NO:39 or a part thereof, wherein the nucleotide sequence of SEQ ID NO:39 is present in IS 6110 gene from a Mycobacterium tuberculosis complex.

13. The probe according to claim 12, which consists of the nucleotide sequence of SEQ ID NO:11.

14. The probe according to claim 11, which contains the nucleotide sequence of SEQ ID NO:27 or a part thereof, wherein the nucleotide sequence of SEQ ID NO:27 is present in cppB gene from a gonococcus.

15. The probe according to claim 14, which consists of the nucleotide sequence of SEQ ID NO:21.

16. The probe according to claim 11, which contains the nucleotide sequence of SEQ ID NO:22 or a part thereof, wherein the nucleotide sequence of SEQ ID NO:22 is present in pLGV440 from a chlamydia.

17. The probe according to claim 16, which consists of the nucleotide sequence of SEQ ID NO:20.

18. The probe according to claim 11, which contains the nucleotide sequence of SEQ ID NO:34 or 35, or a part thereof, wherein the nucleotide sequences of SEQ ID NO:34 and 35 are present in the 5' untranslated region from HCV.

19. The probe according to claim 18, which consists of the nucleotide sequence of SEQ ID NO:34 or 35.

20. The probe according to any one of claims 1 to 19, which is labeled.

21. The probe according to claim 20, which is an oligonucleotide having a nucleotide sequence of continuous 8 to 53 nucleotides from the nucleotide sequence of SEQ ID NO:11, 20, 21, 34 or 35, and which is fluorescence-labeled such that the fluorescence intensity is not repressed if the probe is hybridized to the target nucleic acid, and the fluorescence intensity is repressed if the probe is not hybridized to the target nucleic acid.

22. The probe according to claim 21, which consists of a sequence of continuous 8 or more nucleotides from the nucleotide sequence of SEQ ID NO: 20, 21, 34 or 35.

23. The probe according to claim 22, which is labeled with a rhodamine-type fluorescent dye or an oxazine-type fluorescent dye at the 5' end, and which consists of a sequence of continuous 8 or more nucleotides from the nucleotide sequence of SEQ ID NO:34.

24. The probe according to claim 22, which has a reporter fluorescent dye and a quencher dye as fluorescent dyes for labeling, and which consists of a sequence of continuous 8 or more nucleotides from the nucleotide sequence of SEQ ID NO:11, 20, 21, 34 or 35.

**25.** The probe according to claim 24, wherein the reporter dye is a fluorescein-type dye and the quencher dye is a DABCYL-type dye.

**26.** The probe according to claim 20, which has a label selected from the group consisting of a fluorescent substance, a dye, an enzyme, biotin, gold colloid and a radioisotope.

**27.** A method for detecting a pathogenic microorganism, the method comprising conducting hybridization of the probe defined by any one of claims 1 to 26 to a target nucleic acid from a pathogenic microorganism.

**28.** The method according to claim 27, wherein the hybridization to a target nucleic acid from a pathogenic microorganism is conducted at alkaline pH.

**29.** The method according to 27 or 28, wherein the pathogenic microorganism is a Mycobacterium tuberculosis complex, a gonococcus, a chlamydia or HCV.

**30.** The method according to claim 29, wherein the target nucleic acid from a pathogenic microorganism is IS 6110 gene from a Mycobacterium tuberculosis complex or a fragment thereof.

**31.** The method according to claim 30, wherein hybridization of the probe defined by any one of claims 1, 2, 9 to 11, 12, 13 and 20 to 26 to amplified IS 6110 gene from a Mycobacterium tuberculosis complex and/or a fragment thereof is conducted.

**32.** The method according to claim 31, wherein IS 6110 gene from a Mycobacterium tuberculosis complex and/or a fragment thereof is amplified using a primer having the nucleotide sequence of SEQ ID NO:36 or 37 or a sequence partially overlapping with said sequence.

**33.** The method according to claim 29, wherein the target nucleic acid from a pathogenic microorganism is cppB gene from a gonococcus or a fragment thereof.

**34.** The method according to claim 33, wherein hybridization of the probe defined by any one of claims 3, 4, 9 to 11, 14, 15 and 20 to 26 to amplified cppB gene from a gonococcus and/or a fragment thereof is conducted.

**35.** The method according to claim 34, wherein cppB gene from a gonococcus and/or a fragment thereof is amplified using a primer having the nucleotide sequence of SEQ ID NO:28 or 29 or a sequence partially overlapping with said sequence.

**36.** The method according to claim 29, wherein the target nucleic acid from a pathogenic microorganism is pLGV440 from a chlamydia or a fragment thereof.

**37.** The method according to claim 36, wherein hybridization of the probe defined by any one of claims 5, 6, 9 to 11, 16, 17 and 20 to 26 to amplified pLGV440 from a chlamydia and/or a fragment thereof is conducted.

**38.** The method according to claim 37, wherein pLGV440 from a chlamydia and/or a fragment thereof is amplified using a primer having a nucleotide sequence of any one of SEQ ID NOS:23 to 26 or a sequence partially overlapping with said sequence.

**39.** The method according to claim 29, wherein the target nucleic acid is the 5' untranslated region from HCV or a fragment thereof.

**40.** The method according to claim 39, wherein hybridization of the probe defined by any one of claims 7, 8, 9 to 11, 18, 19 and 20 to 26 to amplified the 5' untranslated region from HCV and/or a fragment thereof is conducted.

**41.** The method according to claim 40, wherein the 5' untranslated region from HCV and/or a fragment thereof is amplified using a primer having a nucleotide sequence of any one of SEQ ID NOS:30 to 33 or a sequence partially overlapping with said sequence.

**42.** A method for detecting a pathogenic microorganism, the method comprising detecting a nucleic acid from a Mycobacterium tuberculosis complex, a gonococcus, a chlamydia or HCV using the method defined by any one of claims 27 to 41.

**43.** A method for detecting a pathogenic microorganism, the method comprising detecting a nucleic acid amplified according to a nucleic acid amplification method which comprises:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3'-terminus or on the 3'-terminal side of the primer; and
(b) incubating the reaction mixture for a sufficient time to generate a reaction product.

**44.** The method according to claim 43, wherein the reaction mixture further contains a chimeric oligonucleotide primer having a sequence substantially homologous to the nucleotide sequence of the nucleic acid as the template.

**45.** The method according to claim 44, wherein the chimeric oligonucleotide primer is represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

**46.** The method according to claim 45, wherein c is 0.

**47.** The method according to claim 45, wherein the nucleotide analog is deoxyriboinosine nucleotide or deoxyribouracil nucleotide and the modified ribonucleotide is ($\alpha$-S) ribonucleotide.

**48.** The method according to claim 43, wherein the chimeric oligonucleotide primer consists of a nucleotide sequence of any one of SEQ ID NOS:13 to 16, 23 to 26 and 28 to 31.

**49.** The method according to any one of claims 27 to 42, which comprises detecting an amplified nucleic acid using the probe defined by any one of claims 1 to 26.

**50.** A chimeric oligonucleotide primer for detecting a pathogenic microorganism represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns).

**51.** The chimeric oligonucleotide primer according to claim 50, wherein c is 0.

**52.** The chimeric oligonucleotide primer according to claim 50, wherein the nucleotide analog is deoxyriboinosine nucleotide or deoxyribouracil nucleotide and the modified ribonucleotide is ($\alpha$-S) ribonucleotide.

**53.** The chimeric oligonucleotide primer according to claim 52, which is represented by any one of SEQ ID NOS:13 to 16, 23 to 26 and 28 to 31.

**54.** A primer for amplifying IS 6110 gene from a Mycobacterium tuberculosis complex and/or a fragment thereof, which has the nucleotide sequence of SEQ ID NO:36 or 37 or a sequence partially overlapping with said sequence.

**55.** A primer for amplifying cppB gene from a gonococcus and/or a fragment thereof, which has the nucleotide sequence of SEQ ID NO:27 or a sequence partially overlapping with said sequence.

**56.** A primer for amplifying pLGV440 from a chlamydia and/or a fragment thereof, which has the nucleotide sequence of SEQ ID NO:22 or a sequence partially overlapping with said sequence.

**57.** A primer for amplifying the 5' untranslated region from HCV and/or a fragment thereof, which has a nucleotide sequence of any one of SEQ ID NOS:41 to 43 or a sequence partially overlapping with said sequence.

**58.** The primer according to any one of claims 54 to 57, which is a chimeric oligonucleotide primer in which a part of the nucleotide sequence is replaced by a ribonucleotide.

**59.** A labeled primer selected from the group consisting of:

(i) a chimeric oligonucleotide primer for detecting a pathogenic microorganism represented by general formula below:

General formula: 5'-dNa-Nb-dNc-3'

(a: an integer of 11 or more; b: an integer of 1 or more; c: 0 or an integer of 1 or more; dN: deoxyribonucleotide and/or nucleotide analog; N: unmodified ribonucleotide and/or modified ribonucleotide, wherein some of dNs in dNa may be replaced by Ns) ;
(ii) the chimeric oligonucleotide primer of (i) wherein c is 0;
(iii) the chimeric oligonucleotide primer of (i) wherein the nucleotide analog is deoxyriboinosine nucleotide or deoxyribouracil nucleotide and the modified ribonucleotide is ($\alpha$-S) ribonucleotide;
(iv) the chimeric oligonucleotide primer of (iii) represented by any one of SEQ ID NOS:13 to 16, 23 to 26 and 28 to 31;
(v) a primer for amplifying IS 6110 gene from a Mycobacterium tuberculosis complex and/or a fragment thereof, which has the nucleotide sequence of SEQ ID NO:36 or 37 or a sequence partially overlapping with said sequence;
(vi) a primer for amplifying cppB gene from a gonococcus and/or a fragment thereof, which has the nucleotide sequence of SEQ ID NO:27 or a sequence partially overlapping with said sequence;
(vii) a primer for amplifying pLGV440 from a chlamydia and/or a fragment thereof, which has the nucleotide sequence of SEQ ID NO:22 or a sequence partially overlapping with said sequence;
(viii) primer for amplifying the 5' untranslated region from HCV and/or a fragment thereof, which has a nucleotide sequence of any one of SEQ ID NOS:41 to 43 or a sequence partially overlapping with said sequence; and
(ix) the primer of any one of (v) to (viii), which is a chimeric oligonucleotide primer in which a part of the nucleotide sequence is replaced by a ribonucleotide.

**60.** The primer according to claim 59, which has a label selected from the group consisting of a fluorescent substance, a dye, an enzyme, biotin and gold colloid.

**61.** A composition for detecting a target nucleic acid, which contains the probe defined by any one of claims 1 to 26.

**62.** A composition for detecting a target nucleic acid, which contains the primer defined by any one of claims 50 to 60.

**63.** The composition according to claim 61 or 62, which is used for detecting a pathogenic microorganism.

**64.** A composition for detecting a pathogenic microorganism, which is used for the method for detecting a pathogenic microorganism defined by claim 43, and which contains at least one reagent for amplifying a target nucleic acid.

**65.** The composition according to claim 64, which contains a reagent selected from the group consisting of a DNA polymerase having a strand displacement activity, an RNase H and a deoxyribonucleotide triphosphate.

**66.** The composition according to claim 65, wherein the DNA polymerase is Bca DNA polymerase lacking 5'$\rightarrow$3' exonuclease from Bacillus caldotenax.

**67.** The composition according to claim 65, wherein the RNase H is a type II RNase H from a bacterium belonging to genus. Pyrococcus and/or a bacterium belonging to genus Archaeoglobus.

**68.** A kit for detecting a pathogenic microorganism, which contains the probe defined by any one of claims 1 to 26.

**69.** A kit for detecting a pathogenic microorganism, which contains the primer defined by any one of claims 50 to 60.

**70.** The kit according to claim 68 or 69, wherein the pathogenic microorganism is a Mycobacterium tuberculosis complex, a gonococcus, a chlamydia or HCV.

**71.** A kit for detecting a pathogenic microorganism, which is used for the method for detecting a pathogenic microorganism defined by claim 27, and which contains at least one reagent for amplifying a target nucleic acid.

**72.** The kit according to claim 71, which contains a reagent selected from the group consisting of a DNA polymerase having a strand displacement activity, an RNase H and a deoxyribonucleotide triphosphate.

**73.** The kit according to claim 72, wherein the DNA polymerase is Bca DNA polymerase lacking 5'→3' exonuclease from Bacillus caldotenax.

**74.** The kit according to claim 72, wherein the RNase H is a type II RNase H from a bacterium belonging to genus Pyrococcus and/or a bacterium belonging to genus Archaeoglobus.

**75.** The kit according to claim 72, which contains a support for capturing an amplification product.

**76.** The kit according to claim 75, wherein the support is selected from the group consisting of a microtiter plate, a bead, a magnetic bead, a membrane and glass.

**77.** A method for detecting a Mycobacterium tuberculosis complex, the method comprising treating a test sample containing a Mycobacterium tuberculosis complex with muramidase to extract a nucleic acid.

Fig. 1

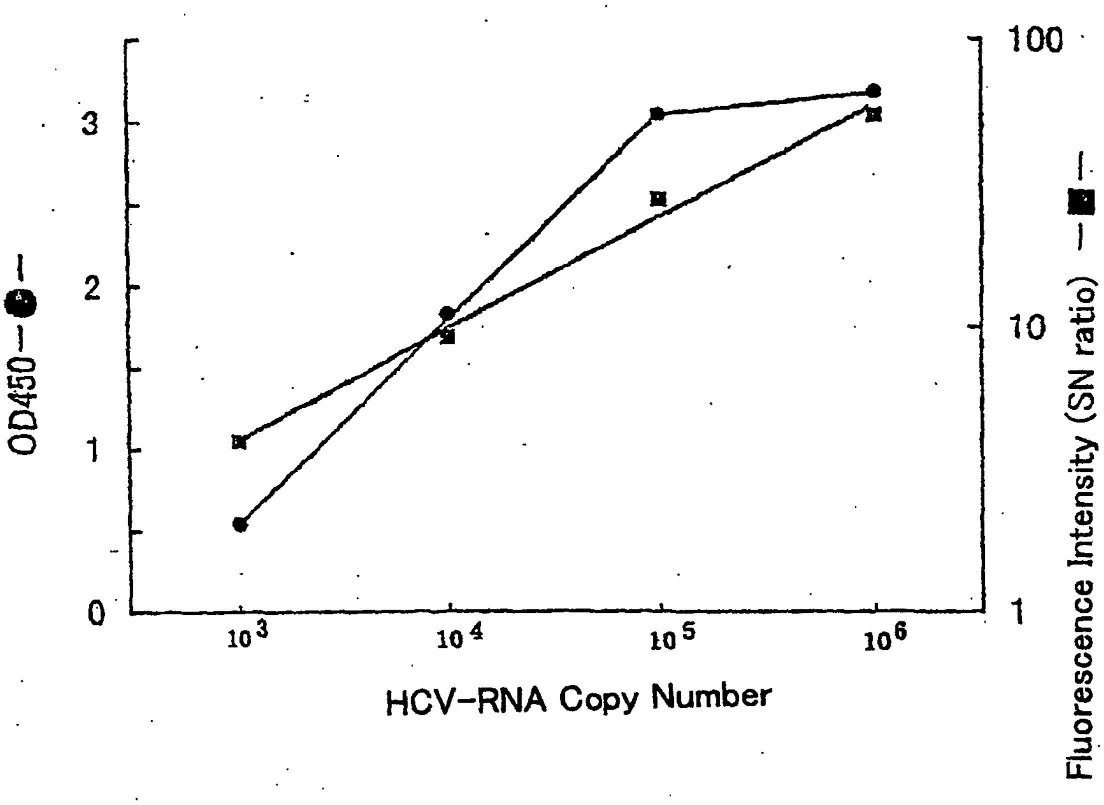
OD450 —●—
0
1
2
3
Fluorescence Intensity (SN ratio) —■—
1
10
100
10 3
10 4
10 5
10 6
HCV-RNA Copy Number

## INTERNATIONAL SEARCH REPORT

International application No.
PCT/JP01/11422

**A. CLASSIFICATION OF SUBJECT MATTER**
Int.Cl[7] C12Q1/68, C12N15/09

According to International Patent Classification (IPC) or to both national classification and IPC

**B. FIELDS SEARCHED**

Minimum documentation searched (classification system followed by classification symbols)
Int.Cl[7] C12Q1/68, C12N15/09

Documentation searched other than minimum documentation to the extent that such documents are included in the fields searched

Electronic data base consulted during the international search (name of data base and, where practicable, search terms used)
Genbank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X | R.A. MCADAM et al., Characterization of a Mycobacterium tuberculosis insertion sequence belonging to the IS3 family. Molecular Microbilogy (1990), Vol.4, No.9, pages 1607 to 1613 | 1,2,12,13, 20-22,24-32, 42,49,61,63, 68,70-76 |
| X | Z.FANG et al., IS6110-Mediated Deletions of Wild-Type Chromosomes of Mycobacterium tuberculosis., Journal of Bacteriology, Feb.1999, Vol.181, No.3, pages 1014 to 1020 | 1,2,12,13, 20-22,24-32, 42,49,61,63, 68,70-76 |
| X | Van EMBDEN et al., Genetic Variation and Evolutionary Origin of the Direct Repeat Locus of Mycobacterium tuberculosis Complex Bacteria, Journal of Bacteriology, May.2000, Vol.182, No.9, pages 2393 to 2401 | 1,2,12,13, 20-22,24-32, 42,49,61,63, 68,70-76 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 March, 2002 (29.03.02) | 09 April, 2002 (09.04.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP01/11422

C (Continuation). DOCUMENTS CONSIDERED TO BE RELEVANT

| Category* | Citation of document, with indication, where appropriate, of the relevant passages | Relevant to claim No. |
|---|---|---|
| X | Peter W.M. HERMANS et al., Insertion Element IS987 from Mycobacterium bovis BDG Is Located in a Hot-Spot Integration Region for Insertion Elements in Mycobacterium turberculosis Complex Strains., Infection and Immunity, Aug.1991, Vol.59, No.8, pages 2695 to 2705 | 1,2,12,13, 20-22,24-32, 42,49,61,63, 68,70-76 |

Form PCT/ISA/210 (continuation of second sheet) (July 1998)

**INTERNATIONAL SEARCH REPORT**

International application No.
PCT/JP01/11422

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
(See extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
In the inventions as set forth in claims 1, 2, 12, 13, 20 to 22, 24 to 32, 42, 49, 61, 63, 68 and 70 to 76, the parts concerning probes of tubercle bacillus

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP01/11422

Continuation of Box No.II of Continuation of first sheet (1)

Although the applicant declares "examples of an appropriate embodiment of the probes according to the invention include those which are stably hybridizable with target nucleic acids in the alkaline region" in page 14 in the description, probes hybridizable in the alkaline regions are described in, for example, JP 8-191700 A and, therefore, cannot be regarded as a "special technical feature".

Also, there have been known various probes and primers of "pathogenic microorganisms" and thus this matter cannot be regarded as a "special technical feature".

*Such being the case, the following 14 groups of inventions are described* in the claims:

(1) in the inventions as set forth in claims 1, 2, 12, 13, 20 to 22, 24 to 32, 42, 49, 61, 63, 68 and 70 to 76, the parts concerning probes of tubercle bacillus;
(2) in the inventions as set forth in claims 3, 4, 14, 15, 20 to 22, 24 to 29, 33 to 35, 42, 49, 61, 63, 68 and 70 to 76, the parts concerning probes of gonococcus;
(3) in the inventions as set forth in claims 5, 6, 16, 17, 20 to 22, 24 to 29, 36 to 38, 42, 49, 61, 63, 68 and 70 to 76, the parts concerning probes of chlamydia;
(4) in the inventions as set forth in claims 7, 18 to 29, 39 to 42, 49, 61, 63, 68 and 70 to 76, the parts concerning probes of HCV;
(5) in the inventions as set forth in claims 8 to 11, the parts concerning probes hybridizable in the alkaline region targeting IS6110;

(6) in the inventions as set forth in claims 8 to 11, the parts concerning probes hybridizable in the alkaline region targeting cppB;
(7) in the inventions as set forth in claims 8 to 11, the parts concerning probes hybridizable in the alkaline region targeting pLGV440;
(8) in the inventions as set forth in claims 8 to 11, the parts concerning probes hybridizable in the alkaline region targeting the 5'-nontranslational region of HCV;
(9) in the inventions as set forth in claims 43 to 48, 50 to 53, 59, 60, 62 to 67, 69 and 70, the parts concerning chimeric oligonucleotide primers;
(10) in the inventions as set forth in claims 54, 58 to 60, 62, 63, 69 and 70, the parts concerning primers of tubercle bacillus;
(11) in the inventions as set forth in claims 55, 58 to 60, 62, 63, 69 and 70, the parts concerning primers of gonococcus;
(12) in the inventions as set forth in claims 56, 58 to 60, 62, 63, 69 and 70, the parts concerning primers of chlamydia;
(13) in the inventions as set forth in claims 57 to 60, 62, 63, 69 and 70, the parts concerning primers of HCV; and
(14) claim 77.

Form PCT/ISA/210 (extra sheet) (July 1998)

SEQUENCE LISTING

<110> Takara Bio, Inc.

<120> Method for detection of virulent organisms

<130> 38-59

<140> EP 01 272 324.3

<141> 2001-07-04

<150> JP 2000-396321

<151> 2000-12-26

<150> JP 2000-396222

<151> 2000-12-26

<150> JP 2001-199552

<151> 2001-06-29

<150> JP 2001-278920

<151> 2001-09-13

<160> 44

<210> 1

<211> 663

<212> DNA

<213> Pyrococcus horikoshii

<400> 1

atgaaggttg ctggagttga tgaagcgggg agggggccgg taattggccc gttagtaatt 60

ggagtagccg ttatagatga gaaaaatatt gagaggttac gtgacattgg ggttaaaga 120

tccaaacaat taactcctgg gcaacgtgaa aaactattta gcaaattaat agatatccta 180

gacgattatt atgttcttct cgttaccccc aaggaaatag atgagaggca tcattctatg 240

aatgaactag aagctgagaa attcgttgta gccttgaatt ctttaaggat caagccgcag 300

aagatatatg tggactctgc cgatgtagat cctaagaggt ttgctagtct aataaaggct 360

gggttgaaat atgaagccac ggttatcgcc gagcataaag ccgatgcaaa gtatgagata 420

gtatcggcag catcaataat tgcaaaggtc actagggata gagagataga gaagctaaag 480

caaaagtatg gggaatttgg ttctggctat ccgagtgatc cgagaactaa ggagtggctt 540

gaagaatatt acaaacaata tggtgacttt cctccaatag ttaggagaac ttgggaaacc 600

gctaggaaga tagaggaaag gtttagaaaa aatcagctaa cgcttgataa attccttaag 660

tga 663

<210> 2

<211> 33

<212> DNA

<213> Artificial Sequence

<220>

<223> PCR primer 1650Nde for cloning a gene encoding a polypeptide having a RNaseHII activity from Pyrococcus furiosus

<400> 2

caggaggaga gacatatgaa aatagggggga att 33

<210> 3

<211> 33

<212> DNA

<213> Artificial Sequence

<220>

<223> PCR primer 1650Bam for cloning a gene encoding a polypeptide having a RNaseHII activity from Pyrococcus furiosus

<400> 3

gaaggttgtg gatccacttt ctaaggtttc tta 33

<210> 4

<211> 672

<212> DNA

<213> Pyrococcus furiosus

<400> 4

atgaaaatag gggaattga cgaagcagga agaggaccag cgatagggcc attagtagta 60

gctactgtcg tcgttgatga gaaaaacatt gagaagctca gaaacattgg agtaaaagac 120

tccaaacaac taacacccca tgaaaggaag aatttatttt cccagataac ctcaatagcg 180

gatgattaca aaatagtgat agtatcccca gaagaaatcg acaatagatc aggaacaatg 240

aacgagttag aggtagagaa gtttgctctc gccttaaatt cgcttcagat aaaaccagct 300

cttatatacg ctgatgcagc ggatgtagat gccaatagat ttgcaagctt gatagagaga 360

agactcaatt ataaggcgaa gattattgcc gaacacaagg ccgatgcaaa gtatccagta 420

gtttcagcag cttcaatact tgcaaaggtt gttagggatg aggaaattga aaaattaaaa 480

aagcaatatg gagactttgg ctctgggtat ccaagtgatc caaaaaccaa gaaatggctt 540

gaagagtact acaaaaaaca caactctttc cctccaatag tcagacgaac ctgggaaact 600

gtaagaaaaa tagaggaaag cattaaagcc aaaaaatccc agctaacgct tgataaattc 660

tttaagaaac ct 672

<210> 5

<211> 224

<212> PRT

<213> Pyrococcus furiosus

<400> 5

Met Lys Ile Gly Gly Ile Asp Glu Ala Gly Arg Gly Pro Ala Ile
1 5 10 15

Gly Pro Leu Val Val Ala Thr Val Val Val Asp Glu Lys Asn Ile
20 25 30

Glu Lys Leu Arg Asn Ile Gly Val Lys Asp Ser Lys Gln Leu Thr
35 40 45

Pro His Glu Arg Lys Asn Leu Phe Ser Gln Ile Thr Ser Ile Ala
50 55 60

Asp Asp Tyr Lys Ile Val Ile Val Ser Pro Glu Glu Ile Asp Asn
65 70 75

Arg Ser Gly Thr Met Asn Glu Leu Glu Val Glu Lys Phe Ala Leu
80 85 90

Ala Leu Asn Ser Leu Gln Ile Lys Pro Ala Leu Ile Tyr Ala Asp
95 100 105

Ala Ala Asp Val Asp Ala Asn Arg Phe Ala Ser Leu Ile Glu Arg
110 115 120

Arg Leu Asn Tyr Lys Ala Lys Ile Ile Ala Glu His Lys Ala Asp
125 130 135

Ala Lys Tyr Pro Val Val Ser Ala Ala Ser Ile Leu Ala Lys Val
140 145 150

Val Arg Asp Glu Glu Ile Glu Lys Leu Lys Lys Gln Tyr Gly Asp
155 160 165

Phe Gly Ser Gly Tyr Pro Ser Asp Pro Lys Thr Lys Lys Trp Leu

170 175 180

Glu Glu Tyr Tyr Lys Lys His Asn Ser Phe Pro Pro Ile Val Arg

185 190 195

Arg Thr Trp Glu Thr Val Arg Lys Ile Glu Glu Ser Ile Lys Ala

200 205 210

Lys Lys Ser Gln Leu Thr Leu Asp Lys Phe Phe Lys Lys Pro

215 220

<210> 6

<211> 626

<212> DNA

<213> Archaeoglobus fulgidus

<400> 6

atgaaggcag gcatcgatga ggctggaaag ggctgcgtca tcggcccact ggttgttgca 60

ggagtggctt gcagcgatga ggataggctg agaaagcttg gtgtgaaaga ctccaaaaag 120

ctaagtcagg ggaggagaga ggaactagcc gaggaaataa ggaaaatctg cagaacggag 180

gttttgaaag tttctcccga aaatctcgac gaaaggatgg ctgctaaaac cataaacgag 240

attttgaagg agtgctacgc tgaaataatt ctcaggctga agccggaaat tgcttatgtt 300

gacagtcctg atgtgattcc cgagagactt tcgagggagc ttgaggagat tacggggttg 360

agagttgtgg ccgagcacaa ggcggacgag aagtatcccc tggtagctgc ggcttcaatc 420

atcgcaaagg tggaaaggga gcgggagatt gagaggctga aagaaaaatt cggggatttc 480

ggcagcggct atgcgagcga tccgaggaca agagaagtgc tgaaggagtg gatagcttca 540

ggcagaattc cgagctgcgt gagaatgcgc tggaagacgg tgtcaaatct gaggcagaag 600

acgcttgacg atttctaaac gaaacc 626

<210> 7

<211> 30

<212> DNA

<213> Artificial Sequence

<220>

<223> PCR primer AfuNde for cloning a gene encoding a polypeptide having a RNaseHII activity from Archaeoglobus fulgidus

<400> 7

aagctgggtt tcatatgaag gcaggcatcg 30

<210> 8

<211> 30

<212> DNA

<213> Artificial Sequence

<220>

<223> PCR primer AfuBam for cloning a gene encoding a polypeptide having a RNaseHII activity from Archaeoglobus fulgidus

<400> 8

tggtaataac ggatccgttt agaaatcgtc 30

<210> 9

<211> 638

<212> DNA

<213> Archaeoglobus fulgidus

<400> 9

catatgaagg caggcatcga tgaggctgga aagggctgcg tcatcggccc actggttgtt 60

gcaggagtgg cttgcagcga tgaggatagg ctgagaaagc ttggtgtgaa agactccaaa 120

aagctaagtc aggggaggag agaggaacta gccgaggaaa taaggaaaat ctgcagaacg 180
gaggttttga aagtttctcc cgaaaatctc gacgaaagga tggctgctaa aaccataaac 240
gagattttga aggagtgcta cgctgaaata attctcaggc tgaagccgga aattgcttat 300
gttgacagtc ctgatgtgat tcccgagaga ctttcgaggg agcttgagga gattacgggg 360
ttgagagttg tggccgagca caaggcggac gagaagtatc ccctggtagc tgcggctta 420
atcatcgcaa aggtggaaag ggagcgggag attgagaggc tgaaagaaaa attcggggat 480
ttcggcagcg gctatgcgag cgatccgagg acaagagaag tgctgaagga gtggatagct 540
tcaggcagaa ttccgagctg cgtgagaatg cgctggaaga cggtgtcaaa tctgaggcag 600
aagacgcttg acgatttcta aacggatccc cggtacc 638

<210> 10

<211> 205

<212> PRT

<213> Archaeoglobus fulgidus

<400> 10

Met Lys Ala Gly Ile Asp Glu Ala Gly Lys Gly Cys Val Ile Gly
1 5 10 15

Pro Leu Val Val Ala Gly Val Ala Cys Ser Asp Glu Asp Arg Leu
20 25 30

Arg Lys Leu Gly Val Lys Asp Ser Lys Lys Leu Ser Gln Gly Arg
35 40 45

Arg Glu Glu Leu Ala Glu Glu Ile Arg Lys Ile Cys Arg Thr Glu
50 55 60

Val Leu Lys Val Ser Pro Glu Asn Leu Asp Glu Arg Met Ala Ala
65 70 75

Lys Thr Ile Asn Glu Ile Leu Lys Glu Cys Tyr Ala Glu Ile Ile
80 85 90

Leu Arg Leu Lys Pro Glu Ile Ala Tyr Val Asp Ser Pro Asp Val

95 100 105

Ile Pro Glu Arg Leu Ser Arg Glu Leu Glu Glu Ile Thr Gly Leu

110 115 120

Arg Val Val Ala Glu HisLys Ala Asp Glu Lys Tyr Pro Leu Val

125 130 135

Ala Ala Ala Ser Ile Ile Ala Lys Val Glu Arg Glu Arg Glu Ile

140 145 150

Glu Arg Leu Lys Glu Lys Phe Gly Asp Phe Gly Ser Gly Tyr Ala

155 160 165

Ser Asp Pro Arg Thr Arg Glu Val Leu Lys Glu Trp Ile Ala Ser

170 175 180

Gly Arg Ile Pro Ser Cys Val Arg Met Arg Trp Lys Thr Val Ser

185 190 195

Asn Leu Arg Gln Lys Thr Leu Asp Asp Phe

200 205

<210> 11

<211> 20

<212> DNA

<213> Artificial Sequence

<220>

<223> Oligonucleotide probe to detect the DNA derived from Mycobacterium tuberculosis.

<400> 11

gacctcacct atgtgtcgac 20

<210> 12

<211> 1378

<212> DNA

<213> Mycobacterium tuberculosis

<400> 12

aggtcggcaa cctgaaccgc cccggcatgt ccggagactc cagttcttgg aaaggatggg 60

gtcatgtcag gtggttcatc gaggaggtac ccgccggagc tgcgtgagcg ggcggtgcgg 120

atggtcgcag agatccgcgg tcagcacgat tcggagtggg cagcgatcag tgagatcgcc 180

cgtctacttg gtgttgctgc gcggagacgg tgcgtaagtg ggtgcgccag gcgcaggtcg 240

atgccggcgc acggcccggg accacgaccg aagaatccgc tgagataaag cgcttgcggc 300

gggacaacgc cgaattgcga agggcgaacg cgattttaaa gaccgcgtcg gctttcttcg 360

cggccgagct cgaccggcca gcacgctaat tacccggttc atcgccgatc atcagggcca 420

ccgcgagggc cccgatggtt tgcggtgggg tgtcgagtcg atctgcacac agctgaccga 480

gctgggtgtg ccgatcgccc catcgaccta ctacgaccac atcaaccggg agcccagccg 540

ccgcgagctg cgcgatggcg aactcaagga gcacatcagc cgcgtccacg ccgccaacta 600

cggtgtttac ggtgcccgca aagtgtggct aaccctgaac cgtgagggca tcgaggtggc 660

cagatgcacc gtcgaacggc tgatgaccaa actcggcctg tccgggacca cccgcggcaa 720

agcccgcagg accacgatcg ctgatccggc cacagcccgt cccgccgatc tcgtccagcg 780

ccgcttcgga ccaccagcac ctaaccggct gtgggtagca gacctcacct atgtgtcgac 840

ctgggcaggg ttcgcctacg tggcctttgt caccgacgcc tacgctcgca ggatcctggg 900

ctggcgggtc gcttccacga tggccacctc catggtcctc gacgcgatcg agcaagccat 960

ctggacccgc caacaagaag gcgtactcga cctgaaagac gttatccacc atacggatag 1020

gggatctcag tacacatcga tccggttcag cgagcggctc gccgaggcag gcatcaacc 1080

gtcggtcgga gcggtcggaa gctcctatga caatgcacta gccgagacga tcaacggcct 1140

atacaagacc gagctgatca aacccggcaa gccctggcgg tccatcgagg atgtcgagtt 1200

ggccaccgcg cgctgggtcg actggttcaa ccatcgccgc ctctaccagt actgcggcga 1260

cgtcccgccg gtcgaactcg aggctgccta ctacgctcaa cgccagagac cagccgccgg 1320

ctgaggtctc agatcagaga gtctccggac tcaccggggc ggttcaggcc ccgatggt 1378

<210> 13

<211> 18

<212> DNA

<213> Artificial Sequence

<220>

<223> Chimeric oligonucleotide primer to amplify the DNA fragment of IS6110 gene derived from Mycobacterium tuberculosis. "nucleotides 16 to 18 are ribonucleotides-other nucleotides are deoxyribonucleotides"

<400> 13

tctcgtccag cgccgcuu 18

<210> 14

<211> 21

<212> DNA

<213> Artificial Sequence

<220>

<223> Chimeric oligonucleotide primer to amplify the DNA fragment of IS6110 gene derived from Mycobacterium tuberculosis. "nucleotides 19 to 21 are ribonucleotides-other nucleotides are deoxyribonucleotides"

<400> 14

gacaaaggcc acgtaggcga a 21

<210> 15

<211> 21

<212> DNA

<213> Artificial Sequence

<220>

<223> Chimeric oligonucleotide primer to amplify the DNA fragment of IS6110 gene derived from Mycobacterium tuberculosis. "nucleotides 19 to 21 are ribonucleotides-other nucleotides are deoxyribonucleotides"

<400> 15

cagtacacat cgatccgguu c 21

<210> 16

<211> 22

<212> DNA

<213> Artificial Sequence

<220>

<223> Chimeric oligonucleotide primer to amplify the DNA fragment of IS6110 gene derived from Mycobacterium tuberculosis. "nucleotides 20 to 22 are ribonucleotides-other nucleotides are deoxyribonucleotides"

<400> 16

gatcgtctcg gctagtgcau ug 22

<210> 17

<211> 46

<212> DNA

<213> Artificial Sequence

<220>

<223> Oligonucleotide primer C4-MT2F-S100 to amplify the DNA fragment of metaroprotease gene from human

<400> 17

gatcgtctcg tccagcgccg cttgataagc actgttcctc cactcc 46

<210> 18

<211> 47

<212> DNA

<213> Artificial Sequence

<220>

<223> Oligonucleotide primer C4-MT2R-A to amplify the DNA fragment of metaroprotease gene from human

<400> 18

gatcggacaa aggccacgta ggcgaagaca cagtcacacc ataaagg 47

<210> 19

<211> 20

<212> DNA

<213> Artificial Sequence

<220>

<223> Oligonucleotide probe to detect the internal control DNA

<400> 19

gcactgttcc tccactccat 20

<210> 20

<211> 19

<212> DNA

<213> Artificial Sequence

<220>

<223> Oligonucleotide prdbe CT1234 to detect the Chramydia criptic plasmid

<400> 20

tcggagtctg agcacccta 19

<210> 21

<211> 20

<212> DNA

<213> Artificial Sequence

<220>

<223> Oligonucleotide probe CppB3 to detect Neisseria gonorrhoeae cppB gene

<400> 21

tccgtaacgt ctctaagtct 20

<210> 22

<211> 218

<212> DNA

<213> Chramydia criptic plasmid

<400> 22

ttgtcttctc gagaagattt atcgtacgca aatatcatct ttgcggttgc gtgtcctgtg 60

accttcatta tgtcggagtc tgagcaccct aggcgtttgt actccgtcac agcggttgct 120

cgaagcacgt gcggggttat cttaaaaggg attgcagctt gtagtcctgc ttgagagaac 180

gtgcgggcga tttgccttaa ccccaccatt tttccgga 218

<210> 23

<211> 22

<212> DNA

<213> Artificial Sequence

<220>

<223> Chimeric oligonucleotide primer to amplify the DNA fragment of Chramydia criptic plasmid "nucleotides 20 to 22 are ribonucleotides-other nucleotides are deoxyribonucleotides"

<400> 23

gtgtcctgtg accttcatta ug 22

<210> 24

<211> 22

<212> DNA

<213> Artificial Sequence

<220>

<223> Chimeric oligonucleotide primer to amplify the DNA fragment of Chramydia criptic plasmid "nucleotides 20 to 22 are ribonucleotides-other nucleotides are deoxyribonucleotides"

<400> 24

ttgtcttctc gagaagattu au 22

<210> 25

<211> 22

<212> DNA

<213> Artificial Sequence

<220>

<223> Chimeric oligonucleotide primer to amplify the DNA fragment of Chramydia criptic plasmid "nucleotides 20 to 22 are ribonucleotides-other nucleotides are deoxyribonucleotides"

<400> 25

tgtgacggag tacaaacgcc ua 22

<210> 26

<211> 22

<212> DNA

<213> Artificial Sequence

<220>

<223> Chimeric oligonucleotide primer to amplify the DNAfragment of Chramydia criptic plasmid "nucleotides 20 to 22 are ribonucleotides-other nucleotides are deoxyribonucleotides"

<400> 26

tccggaaaaa tggtggggtu aa 22

<210> 27

<211> 107

<212> DNA

<213> Neisseria gonorrhoeae

<400> 27

tctgctcgct ttgcttcaat gcctcgttga tatttttccg taacgtctct aagtctgctt 60

tcgtttgttg ctctatgctg gcggcttcgg tgcgtgatgt ctgctcg 107

<210> 28

<211> 20

<212> DNA

<213> Artificial Sequence

<220>

<223> Chimeric oligonucleotide primer to amplify the DNA fragment of Neisseria gonorrhoeae cppB gene. "nucleotides 18 to 20 are ribonucleotides-other nucleotides are deoxyribonucleotides"

<400> 28

ctttgcttca atgcctcguu 20

<210> 29

<211> 17

<212> DNA

<213> Artificial Sequence

<220>

<223> Chimeric oligonucleotide primer to amplify the DNA fragment of Neisseria gonorrhoeae cppB gene "nucleotides 15 to 17 are ribonucleotides-other nucleotides are deoxyribonucleotides"

<400> 29

catcacgcac cgaagcc 17

<210> 30

<211> 21

<212> DNA

<213> Artificial Sequence

<220>

<223> Designed chimeric oligonucleotide primer designated as HCVF to amplify a portion of HCV. "nucleotides 19 to 21 are ribonucleotides-other nucleotides are deoxyribonucleotides"

<400> 30

ctgtgaggaa ctactgtcuu c 21

<210> 31

<211> 18

<212> DNA

<213> Artificial Sequence

<220>

<223> Designed chimeric oligonucleotide primer designated as HCVR3 to amplify a portion of HCV. "nucleotides 16 to 18 are ribonucleotides-other nucleotides are deoxyribonucleotides"

<400> 31

gcagaccact atggcucu 18

<210> 32

<211> 19

<212> DNA

<213> Artificial Sequence

<220>

<223> Designed oligonucleotide primer to synthesize cDNA of HCV.

<400> 32

cactccacca tgaatcact 19

<210> 33

<211> 20

<212> DNA

<213> Artificial Sequence

<220>

<223> Designed oligonucleotide primer to synthesize cDNA of HCV.

<400> 33

ggtgcacggt ctacgagacc 20

<210> 34

<211> 24

<212> DNA

<213> Artificial Sequence

<220>

<223> Designed oligonucleotide probe to detect a DNA fragment amplifying a portion of HCV.

<400> 34

gccaaagcgt ctagccatgg cggg 24

<210> 35

<211> 36

<212> DNA

<213> Artificial Sequence

<220>

<223> Designed oligonucleotide probe to detect a DNA fragment amplyfying a portion of HCV.

<400> 35

gcgagcaaag cgtctagcca tggcgttagt gctcgc 36

<210> 36

<211> 18

<212> DNA

<213> Artificial Sequence

<220>

<223> Oligonucleotide primer to amplify the DNA fragment of IS6110 gene derived from Mycobacterium tuberculosis.

<400> 36

tctcgtccag cgccgctt 18

<210> 37

<211> 21

<212> DNA

<213> Artificial Sequence

<220>

<223> Oligonucleotide primer to amplify the DNA fragment of IS6110 gene derived from Mycobacterium tuberculosis.

<400> 37

gacaaaggcc acgtaggcga a 21

<210> 38

<211> 103

<212> DNA

<213> Artificial Sequence

<220>

<223> A portion of IS6110 gene for amplifying from Mycobacterium tuberculosis

<400> 38

tctcgtccag cgccgcttcg gaccaccagc acctaaccgg ctgtgggtag cagacctcac 60

ctatgtgtcg acctgggcag ggttcgccta cgtggccttt gtc 103

<210> 39

<211> 410

<212> DNA

<213> Artificial Sequence

<220>

<223> A portion of IS6110 gene for amplifying from Mycobacterium tuberculosis

<400> 39

cacagcccgt cccgccgatc tcgtccagcg ccgcttcgga ccaccagcac ctaaccggct 60

gtgggtagca gacctcacct atgtgtcgac ctgggcaggg ttcgcctacg tggcctttgt 120

caccgacgcc tacgctcgca ggatcctggg ctggcgggtc gcttccacga tggccacctc 180

catggtcctc gacgcgatcg agcaagccat ctggacccgc caacaagaag gcgtactcga 240

cctgaaagac gttatccacc atacggatag gggatctcag tacacatcga tccggttcag 300

cgagcggctc gccgaggcag gcatccaacc gtcggtcgga gcggtcggaa gctcctatga 360

caatgcacta gccgagacga tcaacggcct atacaagacc gagctgatca 410

<210> 40

<211> 367

<212> DNA

<213> Artificial Sequence

<220>

<223> A portion of IS6110 gene for amplifying from Mycobacterium tuberculosis

<400> 40

tctcgtccag cgccgcttcg gaccaccagc acctaaccgg ctgtgggtag cagacctcac 60

ctatgtgtcg acctgggcag ggttcgccta cgtggccttt gtcaccgacg cctacgctcg 10

caggatcctg ggctggcggg tcgcttccac gatggccacc tccatggtcc tcgacgcgat 180

cgagcaagcc atctggaccc gccaacaaga aggcgtactc gacctgaaag acgttatcca 240

ccatacggat aggggatctc agtacacatc gatccggttc agcgagcggc tcgccgaggc 300

aggcatccaa ccgtcggtcg gagcggtcgg aagctcctat gacaatgcac tagccgagac 360

gatcaac 367

<210> 41

<211> 73

<212> DNA

<213> Artificial Sequence

<220>

<223> Primer area to amplify a portion of HCV.

<400> 41

cactccacca tgaatcactc ccctgtgagg aactactgtc ttcacgcaga aagcgtctag 60

ccatggcgtt agt 73

<210> 42

<211> 41

<212> DNA

<213> Artificial Sequence

<220>

<223> Primer area to amplify a portion of HCV.

<400> 42

attccggtgt actcaccggt tccgcagacc actatggctc t 41

<210> 43

<211> 53

<212> DNA

<213> Artificial Sequence

<220>

<223> Probe area to detect a DNA fragment amplifying a portion ofHCV .

<400> 43

cgcagaaagc gtctagccat ggcgttagta tgagtgtcgt gcagcctcca gga 53

<210> 44

<211> 60

<212> DNA

<213> Chramydia criptic plasmid

<400> 44

gtgtcctgtg accttcatta tgtcggagtc tgagcaccct aggcgtttgt actccgtcac 60